(19) Europäisches Patentamt
European Patent Office
Office européen des brevets

(11) EP 3 999 094 B1

(12) EUROPEAN PATENT SPECIFICATION

(45) Date of publication and mention of the grant of the patent:
**25.02.2026 Bulletin 2026/09**

(21) Application number: **20742232.0**

(22) Date of filing: **15.07.2020**

(51) International Patent Classification (IPC):
***A61K 38/08*** *(2019.01)* ***A61K 49/00*** *(2006.01)*
***A61P 35/00*** *(2006.01)* ***A61P 29/00*** *(2006.01)*
***G01N 33/50*** *(2006.01)*

(52) Cooperative Patent Classification (CPC):
**A61K 38/08; A61K 49/0032; A61K 49/0056; A61P 29/00; A61P 35/00; G01N 33/5759; G01N 33/582; G01N 33/6893**

(86) International application number:
**PCT/EP2020/070014**

(87) International publication number:
**WO 2021/009237 (21.01.2021 Gazette 2021/03)**

# (54) A UROKINASE PLASMINOGEN ACTIVATOR RECEPTOR-TARGETING PEPTIDE

GEGEN UROKINASE-PLASMINOGENAKTIVATORREZEPTOR GERICHTETES PEPTID

PEPTIDE CIBLANT LE RÉCEPTEUR DE L’ACTIVATEUR DU PLASMINOGÈNE DE TYPE UROKINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2019 SE 1950898**

(43) Date of publication of application:
**25.05.2022 Bulletin 2022/21**

(73) Proprietors:
- **Rigshospitalet**
  **2100 Copenhagen Ø (DK)**
- **University of Copenhagen**
  **1165 Copenhagen K (DK)**
- **Fluoguide A/S**
  **2200 Copenhagen (DK)**

(72) Inventors:
- **KJAER, Andreas**
  **2000 FREDERIKSBERG (DK)**
- **JUHL, Karina**
  **2200 KØBENHAVN N (DK)**
- **KURBEGOVIC, Sorel**
  **2400 KØBENHAVN N (DK)**
- **PLOUG, Michael**
  **2200 KØBENHAVN N (DK)**
- **JØRGEN JENSEN, Knud**
  **2100 KØBENHAVN Ø (DK)**
- **KILDEGAARD SØRENSEN, Kasper**
  **2760 SMØRUMNEDRE (DK)**
- **CHRISTENSEN, Anders**
  **2800 LYNGBY (DK)**
- **ALBRECHTSEN, Morten**
  **2920 CHARLOTTENLUND (DK)**

(74) Representative: **AWA Sweden AB**
**Box 5117**
**200 71 Malmö (SE)**

(56) References cited:
**WO-A1-2016/041558 WO-A2-2006/036071**
**CN-A- 107 652 358 US-B1- 8 568 689**

- **KOHEI SANO ET AL: "Short PEG-Linkers Improve the Performance of Targeted, Activatable Monoclonal Antibody-Indocyanine Green Optical Imaging Probes", BIOCONJUGATE CHEMISTRY, vol. 24, no. 5, 15 May 2013 (2013-05-15), pages 811 - 816, XP055551569, ISSN: 1043-1802, DOI: 10.1021/bc400050k**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the invention

The present invention relates to a uPAR-targeting peptide conjugate with an optimal pharmacokinetic profile intended for administration in a human or animal body. Further, there is provided a uPAR-targeting peptide conjugate and a composition comprising the uPAR-targeting peptide conjugate for use in optical imaging and for diagnosis and/or treatment of a disease.

### Technical Background

Surgery is still the most common method of treatment for cancer diseases. The goal of the cancer surgery is to remove all cancer cells if possible since the best prognosis of cancer treatment comes with complete removal of the cancerous tissue. To achieve this, a certain amount of healthy tissue surrounding the tumor will inexorably be removed as well. All tissue that is removed is carefully examined by microscopy allowing a pathologist judge the extent of the spread and character of the disease. Hence, delineation of margin of the active tumor is a major challenge and usually the location and extent of the tumor spread is mapped preoperatively with computed tomography (CT) or magnetic resonance imaging (MRI). However, the lack of real time imaging tools, low resolution and, for CT, harmful ionization limits these technologies for continuous real time intraoperative imaging, i.e. wherein positron-emission tomography (PET) or CT imaging cannot be performed in a convenient setting when the operation is ongoing.

In contrast, fluorescence imaging enables non-invasive *in vivo* visualization in real time with high temporal and spatial resolution without exposing subjects or personnel in the surgical room to harmful ionizing radiation. Thus, it can be used for safe, simple, and real-time very high-resolution visualization of living organisms and tissue.

Glioblastoma (GBM) is a severe cancer disease with basically no cure and even with aggressive multimodal treatment the median survival is a disappointing 14 months. Accordingly, multidisciplinary efforts are being made to understand the complexity of the disease and find a cure. However, for the last two decades' advances in GBM management have been modest with stagnation in overall survival. Surgery still remains the key treatment and while surgery cannot stand alone, the postoperative survival is determined by the extent of resection. The infiltrative nature of GBM with irregular borders, and tendrils and single cells extending into the apparently healthy tissue makes it difficult to identify clearly and resect completely while at the same time spare healthy adjacent tissue. 5-ALA has recently been approved by the Food and Drug Administration (FDA) for GBM resection and when metabolized in tumors to Protoporphyrin IX it becomes fluorescent with an emission peak at 635 nm. However, the metabolite is not bound to tumor cells and diffuses whereby no clear delineation of the tumor can be seen with 5-ALA and it has also been found low to very-low evidence of 5-ALA improving glioma surgery. Methylene blue, fluorescein sodium, and indocyanine green (ICG) have been approved for human use by the FDA but suffers from short wavelength emissions in the visible spectrum (ICG in the NIR spectrum) and no cancer specificity. This limits the penetration, resolution and contrast expressed as tumor-to-background ratio (TBR) due to autofluorescence, tissue absorbance, scattering, photobleaching and nonspecific uptake in healthy tissue. Thus, new surgical imaging technologies for effective GBM delineation are highly needed.

Targeted molecular fluorescence imaging is such a tool. By combining high affinity targeting molecules with fluorescent dyes, cancer tissue can be specifically targeted and visualized in real time with a high TBR and continuously guide and help the surgeon to distinguish cancerous tissue from healthy tissue intraoperatively.

The urokinase-type Plasminogen Activator Receptor (uPAR) is a well-known cancer target highly expressed in GBMs and several other cancers including, but not limited to, breast cancer, head and neck squamous cell carcinoma, renal cell carcinoma, and lung cancer. The receptor is related to invasion and metastasis, one of the hallmarks of cancer, and the amount of expression is correlated to the aggressiveness of the cancer and poor survival prognosis. Additionally, it is generally highly expressed at the invading front including the activate tumor-stromal microenvironment with chronic inflammation making it an attractive target allowing accurate margin visualization and tumor delineation. For instance, in WO 2016/041558 describes a probe, which is based on a fluorescence-labelled peptide that binds to the uPAR. However, there is still room for improvements and improved methods for cancer resection are needed.

One aim of the present invention is to provide an improved receptor-targeting conjugate for administration before imaging applications and/or surgery in cancer therapy.

### Summary of the invention

The stated purpose above is achieved by a urokinase-type Plasminogen Activator Receptor (uPAR)-targeting peptide conjugate comprising:

- a fluorophore;

- a peptide binding to uPAR; and
- a linker group,

wherein the urokinase Plasminogen Activator Receptor (uPAR)-targeting peptide conjugate has the formula as provided in claim 1. The linker group provides with solubilizing properties of the uPAR-targeting peptide conjugate and provides with enhanced binding properties in relation to the uPAR receptor.

In relation to the above it should be emphasized that the probe shown in WO 2016/041558 is not providing an optimal pharmacokinetic profile or target affinity which compromises optimal and specific contrast in the recorded imaging due to low receptor binding and slow plasma clearance. TBR values for different alternatives are mentioned in WO 2016/041558, however both within and outside the level as provided for the uPAR-targeting peptide conjugate according to the present invention. To summarize, the receptor-targeting conjugate according to the present invention exhibits a novel probe, which provides for an optimal pharmacokinetic profile for certain administration types and indications.

Brief description of the drawings

Fig. 1 illustrates the molecular structure of the uPAR-targeting peptide conjugate according to the present invention, IRDye800CW-AE344.

Fig. 2 illustrates the Surface Plasmon Resonance analysis of the affinity to uPAR of IRDye800CW-AE344, the top line (1) represents IRDye800CW-AE344 : $uPAR^{wt}$ and the bottom line (2) represents AE105 : $uPAR^{wt}$.

Fig. 3 illustrates the spectral analysis of the probe IRDye800CW-AE344 demonstration absorption peak at 777 nm (full line), excitation peak at 784 nm (dotted line), and emission peak at 794 nm (dashed line) resulting in a Stokes shift of 10 nm.

Fig. 4 illustrates the photostability of IRDye800CW-AE344 after continuous laser exposure.

Fig. 5 illustrates the *in* vivo imaging specificity of IRDye800CW-AE344 in an orthotopic GBM. The upper row shows intact brain (NIR image exposure time 500 ms), whereas the lower row shows cross sectioned brain (NIR image exposure time 333 ms), (animal id 029, 6 nmol, 3 h).

Fig. 6 illustrates histology of brain with H&E staining, NIR microscopy, H&E and NIR merged, and immunohistochemical staining of uPAR. The arrow is pointing at a small leptomeningeal metastasis at the basis of the brain and is visible both on H&E staining and NIR microscopy (12 nmol IRDye800CW-AE344, 24h).

Fig. 7 illustrates *ex vivo* dynamic NIR imaging of GBM on cross sectioned brain. The arrows in the pictures indicate the highest TBR (max) for the given dose of IRDye800CW-AE344 (all data are based on images with an exposure time of 333 ms).

Fig. 8 illustrates tumor-to-background ratio for different doses of IRDye800CW-AE344, the lines represents the doses 1mmol (•), 3 nmol ( ▪ ), 6 nmol (▲), and 12 nmol (▼), respectively.

Fig. 9 illustrates tumor mean fluorescence intensities (MFI) for different doses of IRDye800CW-AE344, the different bars represents the doses 1 nmol, 3 nmol, 6 nmol, and 12 nmol, respectively.

Fig. 10 illustrates tumor and background mean fluorescence intensities at 6 nmols IRDye800CW-AE344 in comparison with the TBR, the light gray (left) bar represents tumor, the dark gray (right) bar represents background and the line represents TBR.

Fig. 11 illustrates NIR images of cross sectioned brains at 1 h after injection of (left to right): 3 nmols IRDye800CW-AE344 (active), 3 nM IRDye800CW-AE344 + 600 nmols AE120 (blocked), and 3 nM IRDye800CW-AE354 (mutated).

Fig. 12 illustrates normalized TBRs (ref: active probe). The black bar represents IRDye800CW-AE344 (active), the light gray bar represents AE120 (blocked) and the dark gray bar represents IRDye800CW-AE354 (mutated).

Fig. 13 illustrates images of organs representing the fluorescence intensity and biodistribution of 3 nmols IRDye800CW-AE344 at 1 h post injection (exposure time: 333 ms). The arrow at the small intestines indicates the proximal end. Kidneys oversaturated and thus out of range at the color calibration bar.

Fig. 14 illustrates quantification of fluorescence signal for IRDye800CW-AE344. Left y axis represents the mean fluorescence intensity. Right y axis represents the relative biodistribution. Further, the black bars represent the mean signal intensity (a.u.) and the grey bars represent the relative biodistribution (the data are normalized with the skin as the reference due to highest uptake).

Fig. 15 illustrates images (white light, NIR and merged).

Fig. 16A shows NIR images of cross sectioned brains at 1 h after injection with mean fluorescence intensities in fig. 16B for tumor and background and the corresponding normalized TBR values in fig. 16C.

Fig. 17 there is shown biodistribution and acute toxicity with all data at 1 h post injection.

Specific embodiments of the invention

The invention is defined in the appended set of claims. Below some specific embodiments of the present invention are presented and discussed further.

According to the present invention, the uPAR-targeting peptide conjugate is of the formula:

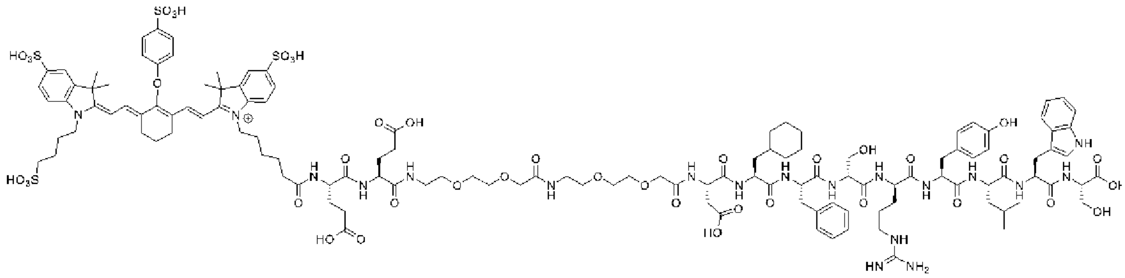

According to another specific embodiment of the present invention, the uPAR-targeting peptide conjugate corresponds to IRDye800CW-Glu-Glu-O2Oc-O2Oc-Asp-Cha-Phe-D-Ser-D-Arg-Tyr-Leu-Trp-Ser-OH. Cha may be interpreted as being β-cyclohexyl-L-alanine.

In another embodiment of the present invention the uPAR-targeting peptide conjugate has a uPAR-binding affinity less than 100 nM, preferably less than 50 nM, preferably less than 25 nM.

As hinted above, the uPAR-targeting peptide conjugate according to the present invention exhibits an optimal pharmacokinetic profile for certain administration types and indications.

With reference to pharmacokinetics, according to one embodiment of the present invention, the uPAR-targeting peptide conjugate is adapted to

- be administered systemically into a human or animal body;
- provide receptor binding at least within 4,500 minutes, preferably 1,200 minutes, more preferably 600 minutes, even more preferably 300 minutes;
- be removed from plasma to make the conjugate bound to the receptor visible, measured as plasma half-life which is the time it takes for the concentration in plasma to be reduced with 50%, and wherein the plasma half-life has a maximum of 4,500 minutes, preferably 1,200 minutes, more preferably 600 minutes, even more preferably 300 minutes;
- provide receptor binding lasting at least 30 minutes;

and wherein the receptor binding affinity, the time it takes to reach the desired receptor binding, the lasting of the receptor binding and plasma half-life translate into a TBR (tumor-to-background ratio) of at least 1.5 and reaches that level within 4,500 minutes, preferably 1,200 minutes, more preferably 600 minutes, even more preferably 300 minutes, after administration into the human or animal body and stays above 1.5 at least 30 minutes after that this level has been obtained.

According to yet another embodiment, the speed of which the protein (P) - ligand (L) complex takes place may be defined as

$$P + L \underset{K_{off}}{\overset{K_{on}}{\rightleftharpoons}} P{\cdot}L$$

where $K_{on}$ is a constant of the binding reaction and where $K_{off}$ is a constant for the dissociation of the protein-ligand complex, and wherein $K_{on} > 1 \times 10^3\ M^{-1}s^{-1}$ and/or $K_{off} < 1 \times 10^{-1}\ s^{-1}$.

According to yet another embodiment, receptor affinity is measured as $IC_{50}$, which is a measurement of the ligand / receptor binding affinity, on 3000 nM or less.

Moreover, according to one specific embodiment, the receptor binding affinity is reached within a time of 30 min measured in vitro.

Furthermore, according to yet another embodiment, a receptor binding lasts at least 120 minutes, preferably at least 300 minutes, after administration into the human or animal body measured using a TBR above 1.5.

According to another aspect, the present disclosure relates to a uPAR-targeting peptide conjugate for use in the treatment of a disease.

According to a further aspect, the present disclosure relates to a uPAR-targeting peptide conjugate for use in optical imaging or fluorescence imaging (FLI) of a disease. Fluorescence imaging enables non-invasive *in vivo* visualization, as well as *in vitro* visualization, in real time with high temporal and spatial resolution without exposing subjects to harmful ionizing radiation. Thus, it can be used for safe, long, and repeated visualization of living organisms and tissue. Accordingly, the uPAR-targeting peptide conjugate of the present disclosure may be administered to a subject in a dose of 0.1-1000 mg per person.

In even a further aspect, the present invention relates to a pharmaceutical composition comprising the uPAR-targeting peptide conjugate together with at least one pharmaceutically acceptable carrier or excipient. The present disclosure also relates to the pharmaceutical composition of the present invention for use in the treatment of a disease. Further, the present disclosure relates to a pharmaceutical composition for use in detection and/or quantification of a disease. Even further, the present disclosure relates to a pharmaceutical composition for use in diagnosis of a disease. The present disclosure also relates to a pharmaceutical composition for use in optical imaging or fluorescence imaging (FLI) of a disease.

In one embodiment of the present disclosure the disease may be selected from the group consisting of cancer and inflammatory diseases. Further, since uPAR is a well-known cancer target highly expressed in GBMs and several other cancers, the cancers that are targeted by the present disclosure may be GBM, including other brain cancers (incl. central and peripheral nervous system), breast cancer, head and neck squamous cell carcinoma and other head and neck cancers (e.g. lip, oral cavity, larynx, nasopharynx, oropharynx, hypopharynx cancers), renal cell carcinoma, lung cancer, colorectum cancer, prostate cancer, stomach cancer, liver cancer, thyroid cancer, bladder cancer, esophagus cancer, pancreas cancer, kidney cancer, corpus uteri cancer, cervix uteri cancer, melanoma, ovary cancer, gallbladder cancer, multiple myeloma, testis cancer, vulva cancer, salivary glands cancer, mesothelioma, penis cancer, kaposi sarcoma, vagina cancer, neuroendocrine tumors and neuroendocrine carcinomas.

In one embodiment of the present disclosure the cancer may be selected from the group consisting of gliomas, glioblastomas or other brain tumors, pancreatic cancer, head-and-neck cancer, breast cancer, lung cancer, colorectal cancer, esophageal cancer, gastric cancer, liver cancer, neuroendocrine tumors, neuroendocrine carcinomas, prostate cancer.

In one embodiment of the present disclosure the cancer is selected from the group consisting of gliomas, glioblastomas, pancreatic cancer, head-and-neck cancer, colorectal cancer, lung cancer and breast cancer. Further, in one specific embodiment of the present disclosure the cancer is gliomas or glioblastomas. In another specific embodiment of the present disclosure the cancer is pancreatic cancer. In even a further specific embodiment of the present disclosure the cancer is breast cancer.

Moreover, also selectivity for cancer tissue is of interest in relation to the present disclosure

According to one specific embodiment of the present disclosure, the receptor-targeting conjugate has a selectivity for cancer tissue of at least 60%, preferably above 70%, more preferably above 80% and most preferably above 90%. Thus, the conjugate product is characterized by having a selectivity for cancer tissue on preferred at least 60%, or 70% or 80%, or 90%. Due to some or all of its properties. With selectivity is understood the relative number of tissues samples removed by the surgeon he/she believes is cancer based on its light intensity/contrast and thereafter confirmed actually is cancer. An example is that the surgeon removed 10 tissues samples that he/she believes is cancer and seven of them is confirmed histologically is cancer given a selectivity of 7/10 - 70%. If 100% of the tissue samples removed by the surgeon believing is cancer are proven to be cancer, the selectivity is 100%. If only half of the tissue samples removed by the surgeon is cancer and the other half is normal tissue the selectivity is 50%.

In another embodiment of the present disclosure the inflammatory diseases are selected from the group consisting of arthritis and atherosclerosis.

In a further aspect there is provided a pharmaceutical composition for use in delineation of cancer tumors or metastases. In even a further aspect there is provided a pharmaceutical composition for use in fluorescence guided tumor or metastasis resection. In this respect the pharmaceutical composition may be administered intravenously, locally or topically. The concentration of the uPAR-targeting peptide conjugate is 0.1-1000 mg per dosage unit. Typically, the concentration of the uPAR-targeting peptide conjugate according to the present disclosure may alternatively be in the ranges of 0.001-1000 mg per dosage unit, 0.01-1000 mg per dosage unit, 0.1-1000 mg per dosage unit, 0.0001-500 mg per dosage unit, 0.001-500 mg per dosage unit, 0.01-500 mg per dosage unit, 0.1-500 mg per dosage unit, 0.0001-100 mg per dosage unit, 0.001-100 mg per dosage unit, 0.01-100 mg per dosage unit, or 0.1-100 mg per dosage unit.

In a further aspect there is provided an optical imaging method comprising the steps of:

(i) administering of a uPAR-targeting peptide conjugate to a target tissue, wherein the uPAR-targeting peptide conjugate comprises a fluorophore, a receptor binding peptide, and a linker group which covalently links the fluorophore to the receptor binding peptide, wherein the receptor binding peptide is binding to a urokinase Plasminogen Activator Receptor (uPAR);

(ii) allowing time for the uPAR-targeting peptide conjugate to accumulate in the target tissue and establishing a

tumor/target tissue-to-background ratio;
(iii) illuminating the target tissue with near infrared light of a wavelength absorbable by the fluorophore;
(iv) detecting fluorescence emitted by the fluorophore and forming an optical image of the target tissue.

As used herein, the term "tissue" refers to isolated *in vitro* and ex *vivo* cells and/or tissues as well as *in vivo* tissues. In one embodiment of the present disclosure the optical imaging method is comprising the step of (i) administering of a uPAR-targeting peptide conjugate to a target tissue *in vivo.* In another embodiment of the present disclosure the optical imaging method is comprising the step of (i) administering of a uPAR-targeting peptide conjugate to a target tissue *in vitro.* The optical imaging method according to the present disclosure provides with a time for the uPAR-targeting peptide conjugate to accumulate in the target tissue to obtain a sufficiently high TBR that is at the most 360 minutes, 180 minutes, 120 minutes, 90 minutes, 60 minutes, 45 minutes, 30 minutes, 15 minutes, 10 minutes or 5 minutes after administration. In a specific embodiment the time for accumulation of the uPAR-targeting peptide conjugate in the target tissue is at the most 60 min, preferably, 45 minutes, preferably 30 minutes, preferably 15 minutes, preferably 10 minutes or preferably 5 minutes after administration. Further, tumor/target tissue-to-background ratio is at least 2.

In one embodiment of the present method the fluorophore has a NIR-light absorption in the range of 700-800 nm. Further, the fluorophore has a NIR-light emission in the range of 750-900 nm. Thus, the uPAR-targeting peptide conjugate is successfully visualized in cancer within the NIR spectrum with improved brightness and photo stability. Further, the uPAR-targeting peptide conjugate of the present invention provides with improved TBR, pharmacokinetics, signal intensity, and increase water solubility.

In one embodiment of the present disclosure the optical imaging method is performed *in vivo.* The optical imaging method may also be performed *in vitro.*

In another aspect there is provided a method for detection of a disease comprising the steps of:

(i) administering of a uPAR-targeting peptide conjugate according to the present invention to a target tissue;
(ii) allowing time for the uPAR-targeting peptide conjugate to accumulate in the target tissue for a set time within the range of 1-120 minutes after administration;
(iii) illuminating the target tissue with near infrared light of a wavelength absorbable by the fluorophore of the uPAR-targeting peptide conjugate;
(iv) detecting fluorescence emitted by the fluorophore of the uPAR-targeting peptide conjugate; and
(v) forming an optical image of the target tissue or quantifying a disease or a combination of both.

In even a further aspect there is provided a use of the uPAR-targeting peptide conjugate or the pharmaceutical composition according to the present invention for optical imaging of cancer. There is also an aspect of the present disclosure that provides with a use of the uPAR-targeting peptide conjugate or the pharmaceutical composition for fluorescence guided tumor or metastasis resection.

In one embodiment of the present disclosure the method for detection of a disease is performed *in vivo.* The method for detection of a disease may also be performed *in vitro.* In one embodiment of the present invention the method for detection of a disease is comprising the step of (i) administering of a uPAR-targeting peptide conjugate to a target tissue *in vivo.* In another embodiment of the present disclosure the method for detection of a disease is comprising the step of (i) administering of a uPAR-targeting peptide conjugate to a target tissue *in vitro.*

Further aspects also relate to a kit of parts for preparing a unit dosage of a uPAR-targeting peptide conjugate preparation, comprising:

- a unit dosage amount of the pharmaceutical composition according to the present invention;
- optionally a unit dosage amount of at least one a pharmacologically active agent;
- optionally a unit dosage amount of an adjuvant;
- optionally a unit dosage of at least one pharmaceutically acceptable carrier or excipient.
- means for separating the unit dosage amounts from each other prior to dispensing; and
- means for dispensing the unit dosage amounts.

In one embodiment the kit of parts according to the present disclosure provides with the unit dosage amount of the pharmaceutical composition in the range of 0.0001-1000 mg. Typically, the unit dosage amount of the pharmaceutical composition according to the present invention may alternatively be in the ranges of 0.001-1000 mg, 0.01-1000 mg, 0.1-1000 mg, 0.0001-500 mg, 0.001-500 mg, 0.01-500 mg, 0.1-500 mg, 0.0001-100 mg, 0.001-100 mg, 0.01-100 mg, or 0.1-100 mg. Further, the unit dosage amounts may be in a total volume range of 0.1-10.0 mL, preferably in a range of 0.1-5.0 mL, preferably a range of 0.5-5.0 mL, preferably a range of 0.5-1.0 mL.

Detailed description of the drawings and examples

Singular references do not exclude a plurality. In the context of the present invention, the terms "a", "an" does not preclude a plurality.

The term "conjugate" means two or more molecules, such as a peptide and a linker and fluorophore, attached to each other by covalent bonds

Key features for an optimal fluorescent probe for surgical guidance include high sensitivity, high specificity, high TBR achieved shortly (1 h) after injection, small dose and yet a high fluorescence signal, easy synthesis with high yield and it has to be well tolerated (non-toxic).

*SPR experiments*

Covalent immobilization of purified human prouPA$^{S356A}$ - -was accomplished by injecting 12.5 μg/ml protein dissolved in 10 mM sodium acetate (pH 5.0) over a CM5 chip that had been pre-activated with NHS/EDC (N-ethyl-N'-[3-diethylamino)propyl]-carbodiimide), aiming at a surface density of >5000 resonance units (RU) corresponding to 100 fmols/mm$^2$. After coupling the sensorchip was deactivated with 1 M ethanolamine. Binding of purified human uPAR as analyte was measured from 4 nM to 0.25 nM at 20°C using 10 mM HEPES, 150 mM NaCl, 3 mM EDTA (pH 7.4) containing 0.05% (v/v) surfactant P20 as running buffer at a flow rate of 50 μl/min. In between cycles the sensorchip was regenerated by two consecutive 10-μl injections of 0.1 M acetic acid/HCl (pH 2.5) in 0.5 M NaCl. The inhibition of 3-fold dilutions of the compounds in question was measured for 4 nM uPAR with identical running conditions. All experiments were performed on a BiacoreT200 instrument.

*Results*

For each inhibition peptide inhibition profile of uPAR binding to immobilized uPA there has been run a preceding standard curve and all calculations are based on the that standard curve. Table 1 summarizes the results.

Table 1.

| | Sequence | $IC_{50}$ uPAR wt | $IC_{50}$ uPAR H47C-N259C |
|---|---|---|---|
| AE105 | D**Cha**FsrYLWS-OH | 7.8 ± 1.0 nM | 4.5 ±1.5 μM |
| AE344 | EE-O2Oc-O2Oc-D**Cha**FsrYLWS-OH | 5.7 ± 0.5 nM | - |
| AE345 | EE-O2Oc-O2Oc-D**Cha**FsrYLWS-$NH_2$ | 31.8 ± 1.5 nM | - |
| AE346 | O2Oc-O2Oc-D**Cha**FsrYLWS-OH | 16.1 ± 0.9 nM | - |
| AE347 | EE-O2Oc-O2Oc-D**Cha**FsrYLWS-$NH_2$ | 5.7 ± 0.5 nM | - |
| AE348 | E-O2Oc-D**Cha**FsrYLWS-$NH_2$ | 6.7 ± 0.2 nM | - |
| AE349 | EE-D**Cha**FsrYLWS-OH | 12.5 ± 0.6 nM | - |
| | ICG-EE- D**Cha**FsrYLWS-OH | 142 ± 13 nM | 0.99 ± 0.05 μM |
| AE353 | IRDye800CW-EE-O2Oc-O2Oc-D**Cha**FsrYLWS-OH | 20.0 ± 1.1 nM | 5.8 ± 0.02 μM |

It is clear from table 1 that a second generation of uPAR targeting peptides have been generated, that are surprisingly better than the original ICG-EE-AE105. In contrast to the ICG derivative, IRDye800CW variant of AE105 (AE353) both targets the wt uPAR with high affinity and show low affinity towards a constrained uPAR variant (negative control). By expanding the hydrophilic linker region, a product with much better solubility properties has been obtained and the original high affinity of the parent peptide (AE105) has been maintained despite having tethered a large reporter group to its N-terminus (IRDye800CW).

*Biochemistry and Optical Properties*

The present invention describes the synthesis of uPAR-targeting fluorescent probe based on a uPAR-targeting peptide conjugate, IRDye800CW-AE344, with the molecular structure shown in fig. 1. The binding properties to uPAR was preserved yielding an $IC_{50}$ = 20 nM ± 1.1 nM (SD) for the competition on the binding of the natural ligand urokinase-type plasminogen activator (fig. 2).

The vis/NIR spectral properties showed an abortion peak at $\lambda_{abs,max}$ = 777 nm (fig. 3) and a slightly right shifted

excitation profile with an excitation peak at $\lambda_{excitation,max}$ = 784 nm. The fluorescence emission spectrum showed peak emission at $\lambda_{emission,max}$ = 794 nm resulting in a Stokes shift of 10 nm. Photostability revealed a preserved fluorescence intensity of 84% after continuous laser exposure for 1h and of 62% after 2h (fig. 4).

*In vivo cancer imaging specificity*

In one example of the present invention fluorescent probe IRDye800CW-AE344 was submitted to *in vivo* cancer imaging. In visual light the orthotopic GBM was non-visible through the intact brain but was clearly visualized on NIR imaging (fig. 5). Additionally, on cross sectioned brain the tumor extent was visible with clear demarcation from healthy tissue allowing distinction between tumor tissue and healthy brain tissue. Histological assessment reveled co-localization of the tumor extent on H&E staining, the NIR microscopy, and on uPAR stained immunohistochemistry (fig. 6) demonstrating that the optical probe of the present invention truly targets the biomarker/tumor with high sensitivity (all tumor is fluorescent) and high specificity (all fluorescent signal is tumor tissue).

For fluorescence-guided surgery (FGS), the surgeon relies on clear identification (signal intensity) and distinction (TBR). The higher dose of 12 nmol revealed a similarly high TBR of 6.7 but at the expense of both delayed peak time of 15 h and a decreased tumor MFI at 58% of that at 6 nmol. Thus, the ideal probe and the optimal dose should lead to both a high intensity and a high TBR.

Compared to prior art, the uPAR-targeting peptide conjugate provides with an improved water solubility, higher signal intensity, and increased TBR. Hence, the fluorescent probe of the present invention safely visualizes GBM with a high TBR of above 4.5 from 1 h to 12 h after injection of 6 nmol that allows for flexible use and complies perfectly with the standard workflow at surgical departments where the probe can be injected shortly prior to surgery as soon as an intravenous access is established e.g. at the preparation for surgery/induction of anesthesia. The useful time-window will be reached when surgery begins and persist throughout even long operations. The highest TBR of 7.0 was observed 3 h post injection of 6 nmol with a high absolute signal intensity. Further, a prolonged incubation time, with the intention to give the fluorescent probe time to clear from circulation and localize in the tumor, would be highly impractical and does not comply with the established clinical workflow and requires the patient to come for an extra visit several days prior to the operation. Also, it is not uncommon that surgery is postponed or cancelled with short notice.

*Dynamic imaging*

In another example, dynamic imaging of orthotopic GBM on cross sections revealed clear tumor visualization at all four doses (1, 3, 6 and 12 nmol). The highest TBR (7.0) was observed 3 h after injection of a dose of 6 nmol (fig. 7). The other doses tested showed maximal TBRs of 4.4 (1 nmol), 6.6 (3 nmol), and 6.7 (12 nmol) at 0.5 h, 1 h, and 15 h, respectively (Fig. 8). The corresponding tumor mean fluorescence intensities (MFI) were 29, 77, 82 and 48 (a.u.), for 1, 3, 6 and 12 nmol doses, respectively (333 ms exposure time). Hence, it was observed a correlation between fluorescence intensity and dose with increasing intensity (both tumor and background) with increasing dose (fig. 9). MFI in both tumor and background were highest at the time of injection and decreased continuously over time. Interestingly, at 6 nmol the TBR initially increased from 4.7 at 1 h to 7.0 at 3h followed by a decrease to 6.1 and 4.5 at 6 h and 12 h, respectively. In the same time there was a continuous decrease in tumor MFI with 108 (a.u.) at 1 h to 82 (a.u.) at 3 h corresponding to a 24% decrease (fig. 10). The background MFI at the same time points decreased from 24 (a.u.) to 11 (a.u.) corresponding to a 54% decrease and the increase in TBR was thus a result of a higher background clearance rate compared to the tumor clearance rate between 1 h to 3 h.

*In vivo Binding Specificity*

Competitively blocking and administration of control ligand (IRDye800CW-AE354; non-binding, scrambled peptide) in animals with orthotopic GBM showed a lower signal intensity compared to the active probe (fig. 11). The normalized TBR values for the groups receiving active, blocked or scrambled probe were 1.00 (reference value), 0.70 ($p$ = 0.006), and 0.52 ($p$ = 0.001), respectively (fig. 12).

*Pharmacokinetics and Toxicology*

At 1 h the kidneys exhibited the highest MFI of 1,236 (a.u.) followed by the lungs, skin, and liver of 101 (a.u.), 99 (a.u.), and 88 (a.u.), respectively. In comparison, the tumor exhibited a signal of 65 (a.u.) and the brain of 19 (a.u.) (fig. 14). The biodistribution showed accumulation primarily in the skin and the kidneys and the normalized uptake in major organs were skin = 100 (reference), kidneys = 38.8, lungs = 4.2, heart = 0.4, spleen = 0.4, liver = 12.2, pancreas = 3.4, colon = 4.0, small intestine = 11.2, ventricle 0.9, brain = 0.7, tumor = 0.1. The signal in the small intestine was limited to the proximal intestines/intestinal content and only modest signal was seen in the distal part (fig. 13 and also shown in fig. 17A).

In fig. 17 there is shown biodistribution and acute toxicity. All data is at 1 h post injection. In 17A, as in fig. 13, there is shown images of organs representing the fluorescence intensity and biodistribution of 3 nmol IRDye800CW-AE344 (exposure time: 333ms). The arrow at the small intestines indicates the proximal end. Kidneys saturated and thus out of range at the color calibration bar. In 17B there is shown liver histology (H&E stained). Moreover, in 17C there is shown kidney histology (H&E stained). Histologically, the liver tissue was normal with lobular configuration without inflammation, fibrosis, cholestasis or deposits. The kidney tissue was normal with preserved glomeruli and tubules without atrophy, infammmation or fibrosis.

Quantification of fluorescence signal (the data are normalized with the skin as the reference) is shown in 17D. In 17E there is shown plasma stability quantified as area under curve (AUC) on HPLC, normalized to 0 hours. The probe was stable within the relevant time window with normalized area under curve (AUC) values of 73% and 67% intact probe after 6 and 12 hours, respectively un murine plasma, and 61% and 43% intact probe after 6 and 12 hours, respectively in human plasma.

*Plasma stability*

1,600 ul human and murine plasma were separately incubated with 2.4 nmol IRDye800-AE344 in 10 ul PBS at 37 °C in dark. 200 ul samples were collected at 0, 0.5, 1, 2, 3, 6, 12 and 24 hours. Plasma proteins were precipitated by addition of 200 ul acetonitrile and the samples were centrifuged at 10,000 G for 10 min. The supernatant was collected for analysis. The supernatant from time zero from both the human and mouse serum was analyzed to establish the retention time of the intact IRDye800CW-AE344 on HPLC-MS on a RSLC Dionex Ultimate 3000 (Thermo) instrument coupled to a QTOF Impact HD. The column was an Aeris widepore 3.6 μm C4 column (150 × 4.6 mm, Phenomenex) and the solvent system was solvent A: water containing 0.1% Formic acid; solvent B: acetonitrile containing 0.1% formic acid. Method: 0-1 min 5% solvent B, 1-18min 5%-50% solvent B with a flowrate of 1 mL/min. This showed that the intact molecule was eluded after 14 minutes. The method, column, and solvents were then transfer to another Dionex Ultimate 3000 (Thermo) which had a 3100-FLD fluorescent detector that employed 774 nm as excitation wavelength and measured the emission at 798 nm. All samples were then run using the settings above and the AUC at the 14 min peak was used to calculate the degradation with 0 h for both murine and human plasma as the reference.

*Fluorescence-guided resection*

Preoperatively, the tumor was visible on WL and NIR images (fig. 15 upper panel). The surgeon performed the resection only assisted by WL until the surgeon considered all tumor tissues was removed. The surgical bed was then evaluated by FLI and the NIR signal revealed residual tumor tissue that was not identified and removed in WL (fig. 15 middle panel). Assisted by NIR, the surgeon identified additional tumor tissue and resected it until no or very little NIR signal was visible indicating complete tumor resection (fig. 15 lower panel). A video of fluorescence-guided surgery is also available in the online supplementary materials.

As should be understood from above, in fig. 15 there is shown fluorescence-guided surgery (6 nmol IRDye800CW-AE344 at 3 h) performed with the EleVision™ IR system. Upper panel: Images were acquired prior to surgery. Middle panel: Pictures were acquired following surgery in white light and clearly visualizes remaining tumor tissue. The signal is more intense compared to upper panel and is due to the fact that the remaining tumor is now more exposed. Lower panel: Images were acquired at the end of the fluorescence-guided surgery and the fluorescent tissue is completely removed indicating complete tumor resection.

*In vivo binding specificity*

Competitive blocking of IRDye800CW-AE344 binding with AE120 (the dimer version of the AE105) and administration of the inactive non-targeting version IRDye800CW-AE354 in orthotopic GBM showed lower signal intensity compared to the active probe (see fig. 16A and 16B). In fig. 16A there is shown NIR images of cross sectioned brains at 1 h after injection of (left to right): 3 nmol IRDye800CW-AE344 (active), 3 nmol IRDye800CW-AE344 + 1.7 mg AE120 (the dimer version of the AE105) (blocked), and 3 nmol IRDye800CW-AE354 (binding inactive). Images are contrast enhanced equally with the scale bar representing the true values. In fig. 16B mean fluorescence intensities for tumor and background are shown.

The corresponding normalized TBR values for the groups receiving active, blocked or inactive probe were 6.6, 4.6 ($p = 0.012$), and 3.4 ($p = 0.0025$), respectively (see fig. 16C).

<u>Experimental</u>

*Biochemistry, Optical Properties and Binding Specificity*

A tumor targeting NIR probe was developed by conjugating the IRDye®800CW fluorophore (LI-COR) with a small uPAR targeting peptide AE105.

The peptide was produced by Fmoc solid-phase peptide synthesis on an automated peptide synthesizer (Biotage® Syro Wave) using a Fmoc Ser(t-Bu) TentaGel S PHB 0.25 mmol/g resin. Subsequently, 5 mg of IRDye®800CW was conjugated to the peptide by HATU/HOAT coupling.

The crude probe was purified by a 3-step process on RP-HPLC (on a Dionex Ultimate 3000 system with a fraction collector). Step 1: Preparative C18 column (Phenomenex Gemini, 110 Å 5 μm C18 particles, 21×100 mm), solvent A, water + 0.1% TFA, solvent B: acetonitrile + 0.1% TFA. Gradient elution (0-5 min: 5%; 5% to 60% 5-32 min) at flow rate 15 mL/min. The fractions containing the fluorescent probe were freeze dried. Step 2: Preparative C4 column (Phenomenex Jupiter, 300Å 5 μm C18 particles, 21×100 mm), solvent A: water + 0.1% TFA, solvent B: Methanol + 0.1% TFA. Gradient elution (0-5 min: 5%; 5% to 60% 5-32 min) at flow rate 15 mL/min. The fractions containing the fluorescent probe were freeze dried. Step 3: Preparative C18 column (Phenomenex Gemini, 110 Å 5 μm C18 particles, 21×100 mm), solvent A: water + 0.1% TFA, solvent B: acetonitrile + 0.1% TFA. Gradient elution (0-5 min: 5% to 30; 30% to 40% 5-40 min) at flow rate 15 mL/min. The fractions containing the fluorescent probe were freeze dried. The product was verified by mass spectrometry and the purity was evaluated by 2-step analytical RP-HPLC.

Fluorophore excitation and emission profiles were obtained with PTI QuantaMaster 400 (Horiba Ltd., Japan). Excitation profile was measured at $\lambda_{emission}$ = 850 nm and the emission profile were measured at $\lambda_{excitation}$ = 740 nm with xenon arc lamps as excitation source. Absorption was measured on a Cary 300 UV-Vis (Agilent, Santa Clara, CA, USA).

The photostability was evaluated by a factor 2 dilution series with four samples from 0.23-1.8 nM IRDye800-AE344 in 100 μL phosphate buffered saline (PBS) placed in a black 96-well plate. The well was placed in a black box with the Fluobeam mounted in the top at a distance of 23 cm from the well plate. Image acquisition was performed at 0 min, 10 min, 15 min, 20 min, 0.5h, 1h, 2h, 3h, 6h, 10h, 15h, 24h. Each dilution sample was normalized to time point 0 and the data from all four samples were pooled.

Surface plasmon resonance (SPR) was applied to determine the $IC_{50}$-value of IRDye800-AE344 on the uPAR•uPA interaction in solution using a Biacore 3000 instrument essentially as described. In brief; pro-$uPA^{S356A}$, which is the natural ligand for uPAR. It is produced recombinantly with the active site S356A mutated so that it has no enzymatic activity (thus, in S356A the active site Ser is replaced by an inactive Ala), was immobilized on a CM5 sensor chip (immobilizing >5000 RU ~ 0.1 pmol pro-uPA/$mm^2$) providing a very high surface density of pro-uPA. This results in a heavily mass transport limited reaction causing the observed association rates ($v_{obs}$) to be directly proportional to the concentrations of binding active uPAR in solution - given only low concentrations of uPAR are tested (here 0.06 nM to 2 nM). The analysis was carried out by measuring vobs of a fixed uPAR concentration (2 nM) incubated with a 3-fold dilution series of IRDye800-AE344 (ranging from 0.076 nM to 1.5 μM) for 300 sec at 20°C with a flow rate of 50 μL/min. A standard curve was measured in parallel (2-fold dilution of uPAR covering 0.06 nM to 2 nM) including one repeated concentration point at the end to validate the biological integrity of the sensor chip. Running buffer contained 10 mM HEPES, 150 mM NaCl, 3 mM EDTA and 0.05 % (v/v) surfactant P20, pH 7.4. The sensor chip was regenerated with two injections of 0.1 M acetic acid, 0.5 M NaCl. The parent nonamer peptide antagonist AE105 (Asp-Cha-Phe-D-Ser-D-Arg-Tyr-Leu-Trp-Ser-OH) was analyzed in parallel as positive control and the closed $uPAR^{H47C-N259C}$ was used as negative control as its uPA binding cavity cannot accommodate AE105.

*Cell Line and Culturing.*

U-87 MG-luc2 cells (Caliper, Hopkinton, MA, USA) were cultured in Dulbecco's Modified Eagle's medium (DMEM) + GlutaMAX added 10% fetal bovine serum (FBS) and 1% Penicillin-Streptomycin at 37 °C in humid 5% $CO_2$ air. The cells were passaged or harvested when reaching 80-90% confluency.

*Animal Models*

All experimental procedures in animals were carried out in accordance with the approval by The Animal Experiments Inspectorate, Denmark. 7-10-week-old female nude mice (strain: Rj:NMRI-Foxn1nu/nu, JANVIER LABS, France) were orthotopically xenografted with U-87 MG-luc2 cells.

Prior to any surgical procedure, the animals were anesthetized with Hypnorm (0.315 mg/ml fentanyl, 10mg/ml fluanisone) + Midazolam (5mg/ml) + sterile water in the ratio 1:1:2 by subcutaneous injection of 0.01 ml/g body weight of the solution.

The orthotopic GBM tumor model was established by inoculation of 500.000 cells in 10μL ice cold PBS in the right hemisphere (1.5 mm lateral and 0.5 mm posterior to the bregma at 2 mm depth) using a stereotaxic frame (KOPF INSTRUMENTS, Tujunga, CA, USA) with the automated microinjection pump UMP3 (WPI, Sarasota, FL, USA). The cells were injected over 5 min with the syringe kept in place for further 3 min before retraction. Tumor growth was subsequently

monitored on MRI with the BioSpec 7T (Bruker, Billerica, MA, USA) with axial and coronal T2-wighted sequences. When the tumor size reached 2-17 $mm^3$, the animals were included in the fluorescence imaging protocol.

*Fluorescence Imaging*

All image acquisition was performed with the Fluobeam system with (Fluosoft version: 2.2.1) (Fluoptics, Grenoble, France). To characterize the *in vivo* biodistribution and tumor imaging properties, IRDye800-AE344 was administered through tail vein injection into all the GBM bearing mice (n = 35) at four different doses: 1 nmol, 3 nmol, 6 nmol, and 12 nmol. Two mice were sacrificed for each time point and the brain was removed and cross sectioned through the tumor for imaging. Preliminary testing revealed slower background clearance for the higher doses prolonging the time window. Accordingly, image acquisition was performed at following time points after injection:

- 1 nmol: 0.5 h, 1 h, and 2 h
- 3 nmol: 1 h, 2 h, 3 h, and 5 h
- 6 nmol: 1 h, 3 h, 6 h, and 12 h
- 12 nmol: 1 h, 3 h, 5 h, 10 h, 15 h, and 24 h

The target specificity to uPAR was evaluated by two different methods: competitive blocking with the uPAR targeting peptide AE120 ($(DChaFsrYLWSG)_2$-βAK) and IRDye800 conjugated to a scrambled peptide, AE354 (IRDye800CW-Glu-Glu-NH-$CH_2$-$CH_2$-O-$CH_2$-$CH_2$-O-$CH_2$-CO-NH-$CH_2$-$CH_2$-O-$CH_2$-$CH_2$-O-$CH_2$-CO-Asp-Cha-Glu-(D) Ser-(D)Arg-Tyr-Leu-Glu-Ser-OH) with similar peptide length as the active peptide. The competitive blocking dose of 1.4-2.8 mg AE120 was injected intraperitoneally 15-30 min prior to intravenous injection of 3 nmol IRDye800CW-AE344 and imaged at 1 h (n=5). The scrambled peptide was administered at 3 nmol and the animals were imaged after 1 h (n=4).

Biodistribution was assessed in animals (n=2) receiving 3 nmol IRDye800-AE344 and was euthanized after 1h for organ dissection and imaging (exposure time: 333 ms). Due to renal excretion, the fluorescence signal in the kidneys were out of scale compared to all the other organs. Thus, images were acquired at a lower exposure time of 40 ms and the signal intensity was subsequently extrapolated to be comparable to the other organs. The skin was imaged partially (1.25 g) and the biodistribution was extrapolated with respect to the full weight of the skin (4.9 g).

*Image Processing and Analysis*

Images were analyzed and processed in ImageJ 1.52a, NIH, USA. Signal measurements were performed on the raw images generated by the Fluoptics system. Tumor signal was measured as a mean fluorescence intensity of the whole tumor and background signal was measured as the mean of a representative area with no tumor on the contralateral hemisphere. Presented pictures are contrast enhanced in ImageJ with the Contrast Enhancement (Saturated pixels: 0.3%).

*Pathological Assessment*

Pathological assessment was used to evaluate the colocalization of the fluorescence signal and cancer cells (sens+spec). The cross sectioned brain specimens from the fluorescence imaging were either paraffin embedded for H&E and IHC staining, or cryostat sectioned for fluorescence microscopy. Paraffin embedding was performed by fixation in 4 % formalin for 24 h following suspension in ethanol and subsequent paraffine embedding. The embedded tissue was axially sectioned into xx um thick slices and stained. IHC staining was performed with an in-house antibody, poly-rabbit-antihuman-uPAR, produced by Finsen Laboratory, Rigshospitalet (Copenhagen, Denmark) and H&E staining was performed by common standard procedure. The stained slides were imaged with the ZEISS Axio Scan.Z1 slide scanner (Carl Zeiss, Oberkochen, Germany).

Cryostat sectioning was performed by fixation of the specimen in Tissue-Tek O.C.T. on dry ice. The fixed tissue was sliced axially and mounted on slides for immediate fluorescence imaging.

Liver and kidneys: Tissue was formalin fixed and paraffin embedded. Sections of 2-4 um thick were cut and a routine staining panel applied including: H&E, modified sirius, PAS and Masson trichome for both and additional PAS with silver for kidneys and PAS with diastase, iron, reticulin artisan and oxidised orcein for the livers. Livers were immunohistochemically stained, immunohistochemical evaluation on 3 μm thick sections was done using the CK7 antibody from Dako/Agilent, GA619 (clone OV-TL12/30) following the manufacturer's instructions. The staining took place on the Omnis from Agilent utilizing the EnVision Flex+ detection kit (GV800). The primary antibody was diluted using Antibody Diluent (Dako DM830) and were incubated for 20 minutes. The sections were counterstained with hematoxylin.

*Materials and procedure for peptide synthesis*

*Materials*

IRDye800-Glu-Glu-O2Oc-O2Oc-Asp-Cha-Phe-D-ser-D-arg-Tyr-Leu-Trp-Ser-OH. All other materials were obtained from commercial suppliers; Fmoc Ser(t-Bu) TentaGel S PHB 0.25 mmol/g, was from Rapp Polymere GmbH. All Amino acids were Fmoc Nα-amino protected and carried side-chain protecting groups: tert-butyl (Ser, Asp, Glu and Tyr), tert-butyloxycarbonyl (Boc, for Trp), 2,2,4,6,7-pentamethyl-dihydrobenzofuran-5-sulfonyl (Pbf, for Arg). Fmoc-O2Oc-OH Fmoc-[2-(2-aminoethoxy)ethoxy]acetic acid N,N-dimethylformamide (DMF), N-methylpyrrolidone (NMP),N-[(1H-benzotriazol-1-yl)(dimethylamino)methylene]-N-methylmethanaminium hexafluorophosphate N-oxide (HBTU), 1-Hydroxy-7-azabenzotriazole (HOAt), trifluoroacetic acid (TFA), piperidine and N,N-diisopropylethylamine (DIPEA) were from Iris Biotech GmbH, while methanol, acetonitrile, formic acid, triethylsilane (TES), dichloromethane (DCM), 1-[Bis(dimethylamino)methylene]-1H-1,2,3-triazolo[4,5-b]pyridinium 3-oxide hexafluorophosphate, N-[(Dimethylamino)-1H-1,2,3-triazolo-[4,5-b]pyridin-1-ylmethylene]-N-methylmethanaminium hexafluorophosphate N-oxide (HATU) were from Sigma-Aldrich. IRDye®800CW Carboxylate from LI-COR.

*Peptide synthesis*

The peptide was produced by Fmoc solid-phase peptide synthesis on an automated peptide synthesizer; Biotage® Syro Wave. The synthesis was carried out on a Fmoc Ser(t-Bu) TentaGel S PHB 0.25 mmol/g resin, using 0.1 mmol scale.

The $N^{\alpha}$Fmoc deprotection was performed at room temperature (RT) in two stages by treating the resin with piperidine/DMF (2:3) solution for 3 min, followed by piperidine/DMF (1:4) solution for 15 minutes. The resin was then washed with NMP (x3), DCM (x1), and then NMP (x2). All couplings of amino acids used 4 eq. of amino acid and O2Oc spacer, 4eq. of HOAt, 3.9 eq. of HBTU, and 7.4 eq. of DIEA in NMP. The coupling time was 60 minutes at RT. All couplings were repeated to ensure maximum incorporation, and after the second coupling the resin was washed with NMP (x4).

When all amino acids and O2Oc spacer was attached and the last Fmoc group was removed, a test cleavage was performed, which showed that the purity was above 80%, calculated from the LC-MS chromatogram. In order to attach the fluorophore to the synthesized peptide, 5 mg of IRDye®800CW Carboxylate was dissolved in 1ml DMF. To this 2 mg of HATU, 1 mg HOAT and 1.7 μl DIEA was added. The solution was shaken for 5 min and then transferred to 100 mg of resin with the synthesized peptide and left to react for 12 hours in darkness. After end reaction the resin was washed with DMF(x5) and DCM(x6). Then the peptide was cleaved from the resin using 95 % TFA; 5% Water with a 2 hour reaction time. The TFA was removed with nitrogen flow. The peptide was then precipitated in cold diethyl ether.

*Purification*

The crude peptide was purified by a 3 step process on RP-HPLC (on a Dionex Ultimate 3000 system with an fraction collector). First the peptide was purified on a preparative C18 column (Phenomenex Gemini, 110 Å 5 μm C18 particles, 21×100 mm) using the following solvent system: solvent A, water containing 0.1% TFA; solvent B, acetonitrile containing 0.1% TFA. Gradient elution (0-5 min: 5%; 5% to 60% 5-32 min) was applied at a flow rate of 15 mL $min^{-1}$. The fractions containing the fluorescent peptide were freeze dried. They were purified using the following conditions on step 2: preparative C4 column (Phenomenex jupiter, 300Å 5 μm C18 particles, 21×100 mm) using the following solvent system: solvent A, water containing 0.1% TFA; solvent B, Methanol containing 0.1% TFA. Gradient elution (0-5 min: 5%; 5% to 60% 5-32 min) was applied at a flow rate of 15 mL $min^{-1}$. The fractions containing the fluorescent peptide were freeze dried. And then final step of the purification performed on a preparative C18 column (Phenomenex Gemini, 110 Å 5 μm C18 particles, 21×100 mm) using the following solvent system: solvent A, water containing 0.1% TFA; solvent B, acetonitrile containing 0.1% TFA. Gradient elution (0-5 min: 5% to 30; 30% to 40% 5-40 min) was applied at a flow rate of 15 mL $min^{-1}$.

*Analysis*

Peptide purity was established using 2 different analytical methods performed on UHPLC-MS on a RSLC Dionex Ultimate 3000 (Thermo) instrument coupled to a QTOF Impact HD. During the first method an Aeris 3.6 μm widepore C4 column (50 × 2.1 mm, Phenomenex) with a flow rate of 0.5 mL/min was used with the following solvent system: solvent A, Water containing 0.1% Formic acid; solvent B, Methanol containing 0.1% Formic acid. The column was eluted using a linear gradient from 5%-75% of solvent B.

The second method involved kinetex 2.6 μm EVO 100 Å C18 column (50 × 2.1 mm, Phenomenex) with a flow rate of 0.5 mL/min. The following solvent system was used: solvent A, Water containing 0.1% Formic acid; solvent B, acetonitrile containing 0.1% Formic acid. The column was eluted using a linear gradient from 5%-100% of solvent B. The synthesis yielded 2 mg of 98% pure peptide. Chemical Formula: $C_{129}H_{173}N_{18}O_{41}S_4$. Calculated Mass 2758.0888; found: $[M+2H]^{2+}$ 1380.0523; $[M+3H]^{3+}$ 920.3723; $[M+4H]^{4+}$ 690.5289

Discussion

Key features for an optimal fluorescent probe well-suited for surgical guidance are 1) high sensitivity and specificity, 2) high TBR achieved within few hours, 3) need of a low dose to yield high fluorescence signal, and 4) a favorable safety profile. In the present study, we successfully synthesized a new peptide-based fluorescent probe that effectively visualized GBM *in vivo* with a TBR above 4.5 between 1 to 12 hours after an injection dose of 6 nmol. This allows for flexible use and complies with the standard workflow at surgical departments where the probe can be injected shortly before surgery, e.g. at the preparation for surgery or induction of anesthesia. The useful time-window starts when the surgery begins and persist throughout even long procedures. The use of a small peptide-based probe thus seems particularly applicable for clinical intraoperative imaging. Accordingly, the inventors have showed that fluorescence-guided resection of a human orthotopic GBM xenograft could be performed in a mouse as early as 3 hours after administration of 6 nmol IRDye800CW-AE344.

In contrast, antibody-based probes circulate in the blood for a long time (days) due to the large molecular size impairing high TBR at an early time point due to a high background signal lasting for days. Only by waiting days after injection can a sufficiently high TBR be obtained. An antibody-based fluorescent probe, IRDye800CW-Cetuximab, with a reported blood half-life of approximately 24h has been tested in humans with GBM and it needed to be infused 3 days prior to surgery (28, 29). In the view of the inventors, this may be impractical and does not comply with the established clinical workflow and requires the patient to come for an extra visit several days prior to the operation. Also, it is not uncommon that surgery is postponed or cancelled with short notice and in these cases, the antibody-based probe would already have been injected and thus be of no use. Injected antibodies, in general, also carries a well-known risk of side effects, especially immune-related adverse events. The use of antibody-based probes for fluorescence-guided surgery therefore seems quite inconvenient from a clinical perspective limiting their applicability and might not be the most obvious approach. Different strategies have been pursued to reduce the size while at the same time keep the high binding affinity properties of antibodies to improve the pharmacokinetic profile. This has resulted in different smaller antibody fragments, e.g. nanobodies and affibodies, with high binding affinity and to some degree a reduced circulation time.

It is expected that the new probe(s) according to the present invention are well tolerated and safe also in humans. This is based on the fact that its components have previously been used in humans with no safety issues. First, our binding moiety has previously been tested as a uPAR targeting PET probe ($^{68}$Ga-NOTA-AE105) in multiple cancer types (14, 34) (NCT03278275, NCT02755675, NCT03307460, NCT02960724, NCT02805608, NCT02964988, NCT02681640, NCT02965001) including an ongoing phase II trial in patients with GBM (NCT02945826). In total, more than 400 patients have so far been scanned and no adverse events or toxicity has been observed suggesting a favorable safety profile with no observed on or off target toxicity. Secondly, IRDye800CW is a novel fluorophore that has been tested safe in humans although not as extensively as the traditional fluorophore, ICG that has been proven safe on a larger scale. However, IRDye800CW has superior optical and pharmacokinetic properties with increased brightness, less bleaching and rapid renal clearance. Currently, at least 10 clinical trials have been performed with IRDye800CW labelled probes. Most of these studies are with IRDye800CW labeled antibodies and only one study has been with a labeled small peptide. There has been no reporting on severe adverse events and the reported mild side effects are most likely akin to antibody reactions. Therefore, taken together and on the basis of the safety profiles of the uPAR-targeting peptide and IRDye800CW, separately, one can reasonably assume that the safety profile of the two compounds combined are also safe for human use. In support of this, our pathological assessment of the kidneys and the liver showed no pathological findings indicating no toxicity in these organs, especially the kidney where the probe accumulates and is cleared. Furthermore, IRDye800CW has spectral properties similar to ICG enabling compatibility with preexisting imaging equipment designed for ICG, which is key for a successful translation and clinical implementation.

## Claims

**1.** A urokinase Plasminogen Activator Receptor (uPAR)-targeting peptide conjugate comprising:

- a fluorophore;
- a peptide binding to uPAR; and
- a linker group,

wherein the urokinase Plasminogen Activator Receptor (uPAR)-targeting peptide conjugate has the formula

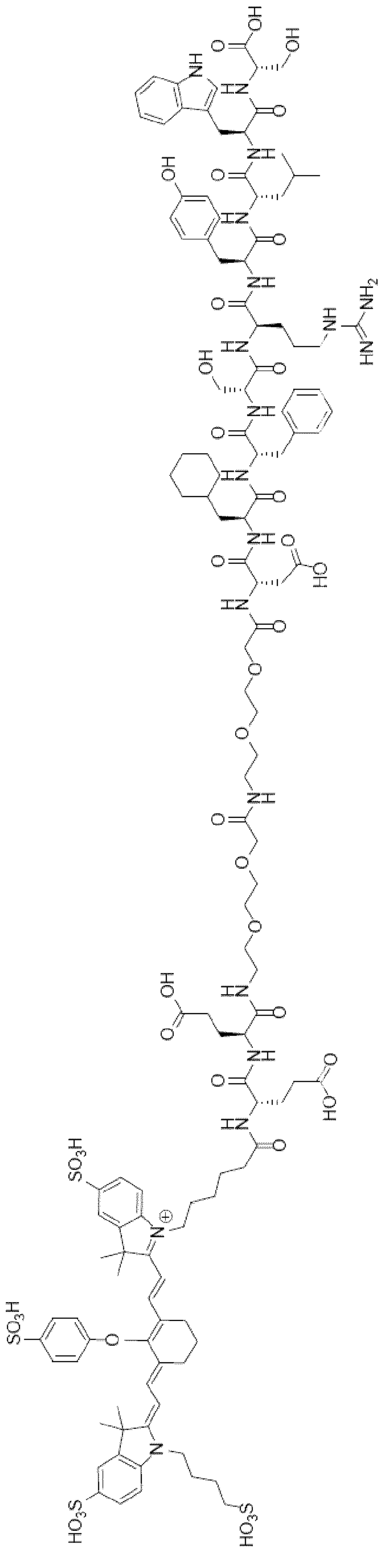

**2.** A pharmaceutical composition comprising the uPAR-targeting peptide conjugate according to claim 1 together with at

least one pharmaceutically acceptable carrier or excipient.

## Patentansprüche

1. Auf Urokinase-Plasminogen-Aktivator-Rezeptor(uPAR) zielendes Peptidkonjugat, umfassend:

   - ein Fluorophor;
   - ein Peptid, das an uPAR bindet; und
   - eine Linkergruppe,

   wobei das auf Urokinase-Plasminogen-Aktivator-Rezeptor(uPAR) zielende Peptidkonjugat die Formel

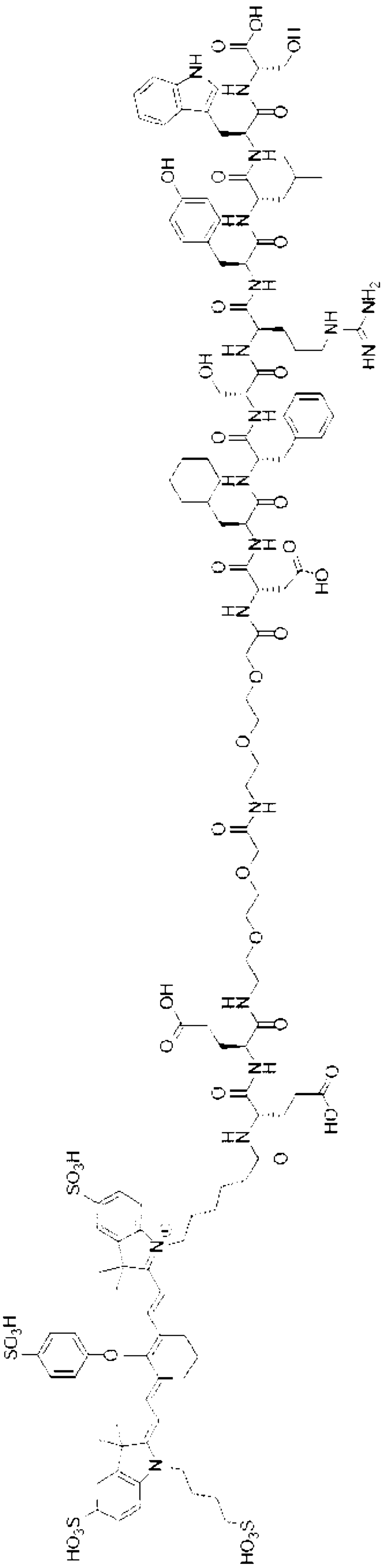

aufweist.

**2.** Pharmazeutische Zusammensetzung, umfassend das auf uPAR zielende Peptidkonjugat nach Anspruch 1 zusammen mit mindestens einem pharmazeutisch unbedenklichen Träger oder Exzipienten.

## Revendications

**1.** Conjugué récepteur de l'activateur du plasminogène d'urokinase (uPAR)-peptide de ciblage comprenant :

- un fluorophore ;
- un peptide se liant à l'uPAR ; et
- un groupe lieur,

dans lequel le conjugué récepteur de l'activateur du plasminogène d'urokinase (uPAR)-peptide de ciblage a la formule

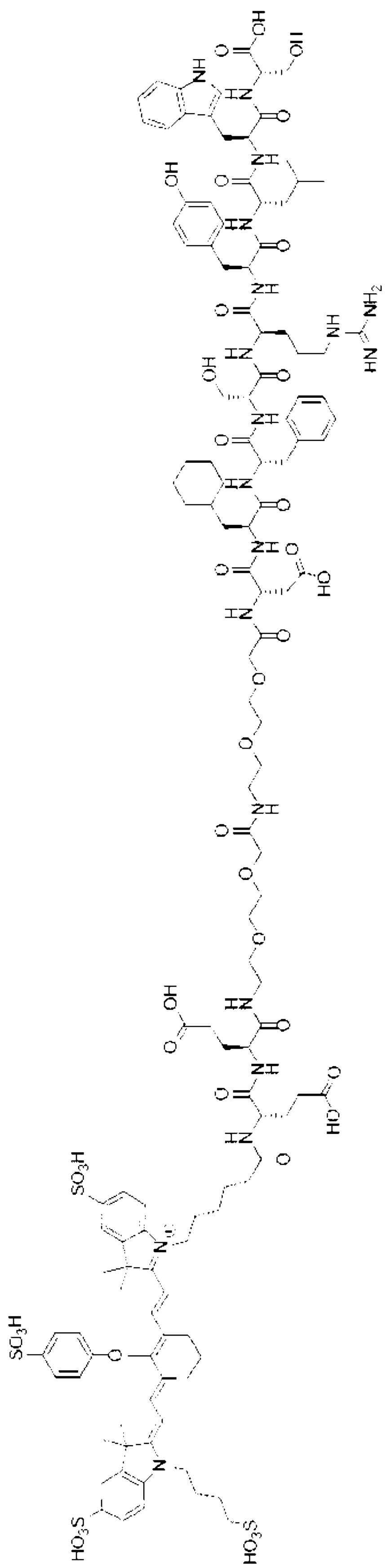

.

**2.** Composition pharmaceutique comprenant le conjugué uPAR-peptide de ciblage selon la revendication 1 conjointement avec au moins un support ou excipient pharmaceutiquement acceptable.

Fig. 1

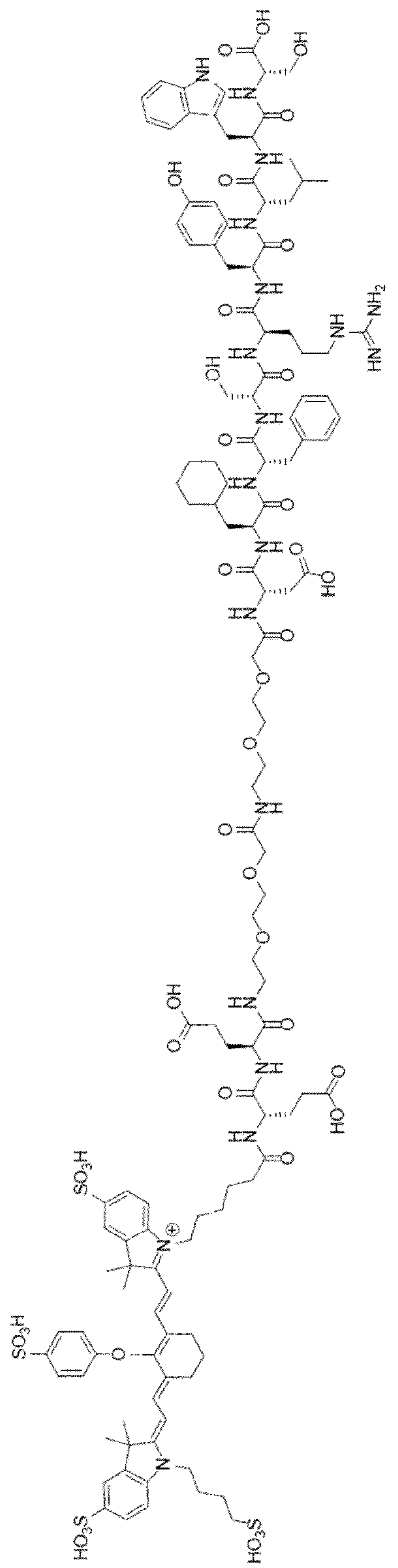

Fig. 2

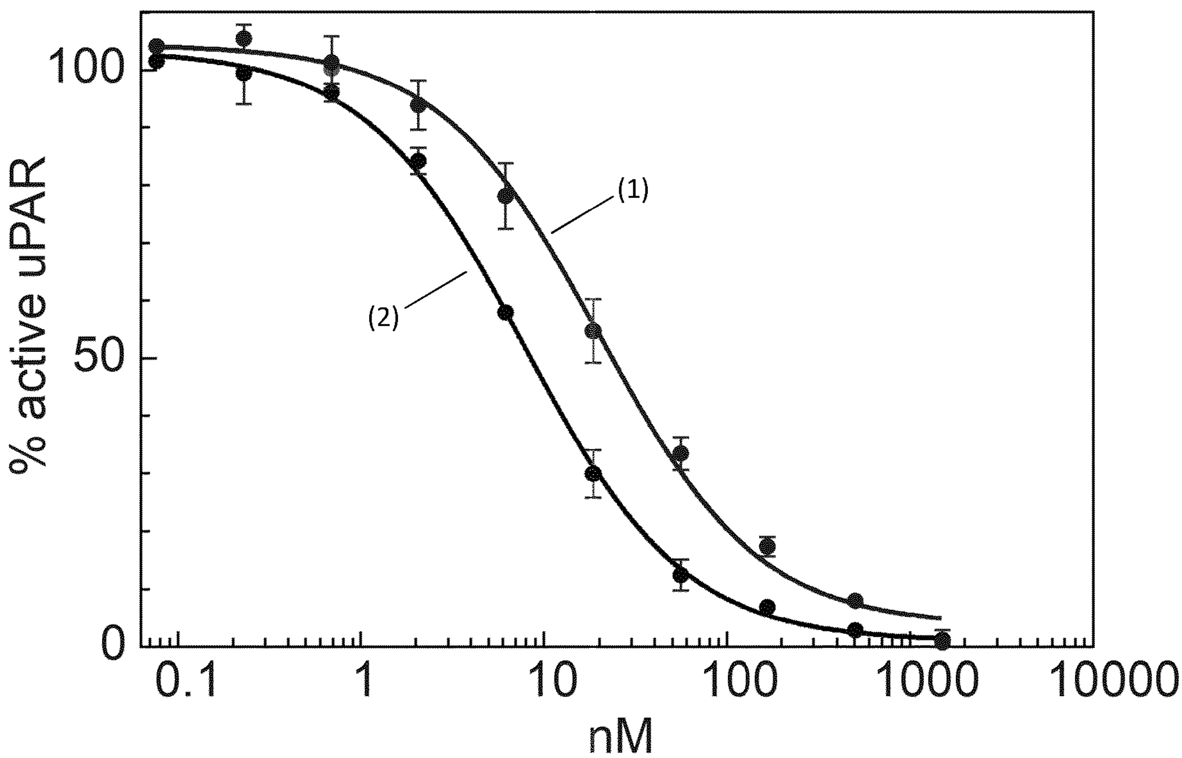

Fig. 3

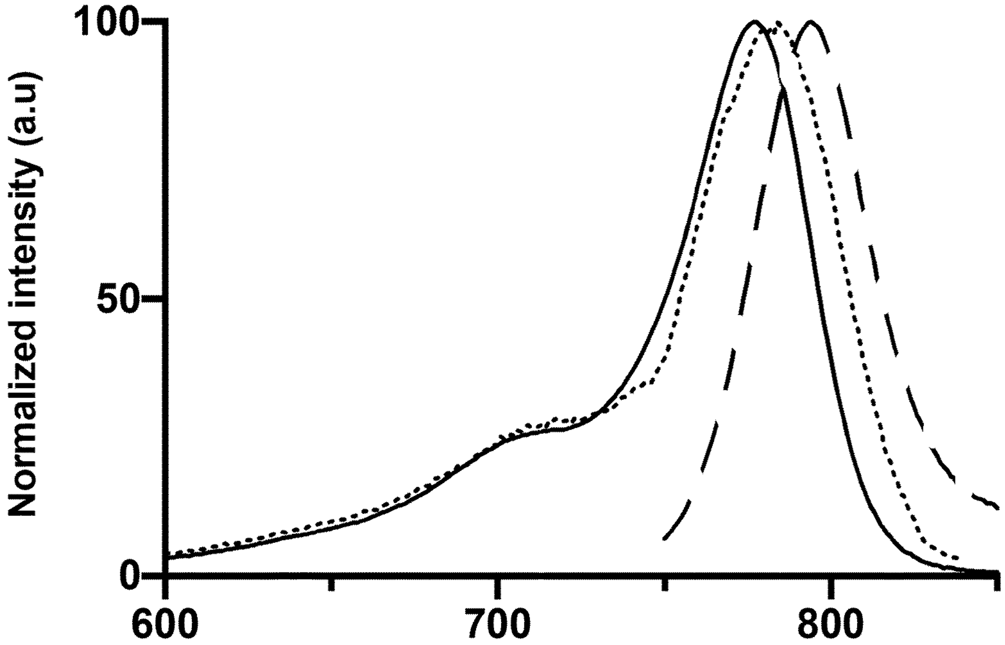
100
50
0
Normalized intensity (a.u)
600
700
800

Fig. 4

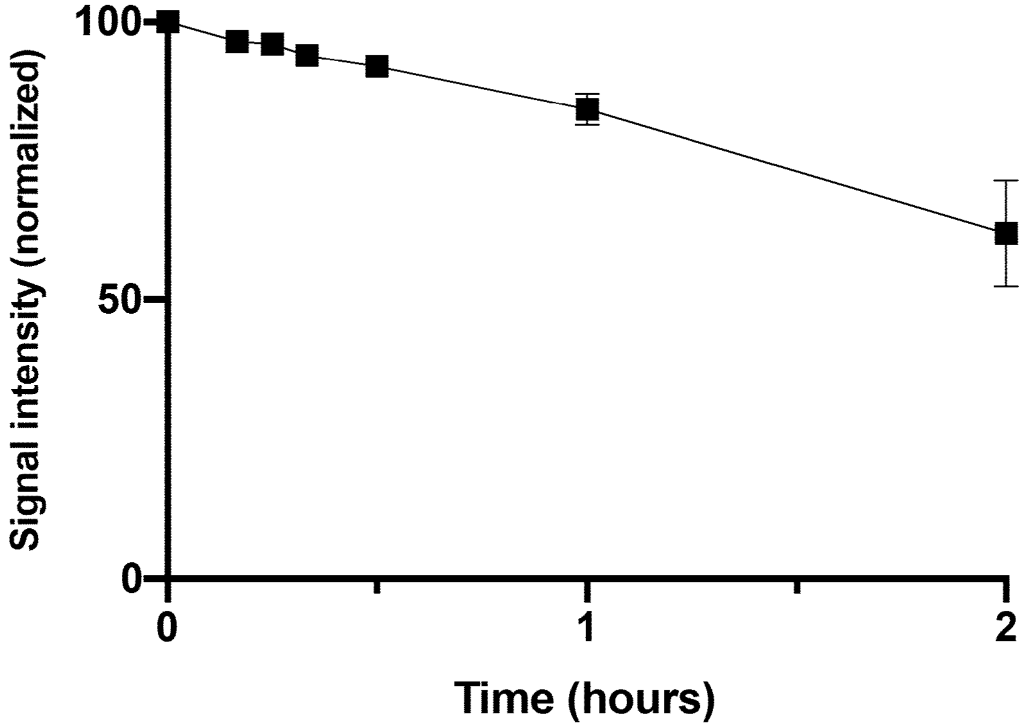
100
50
0
Signal intensity (normalized)
0
1
2
Time (hours)

Fig. 5

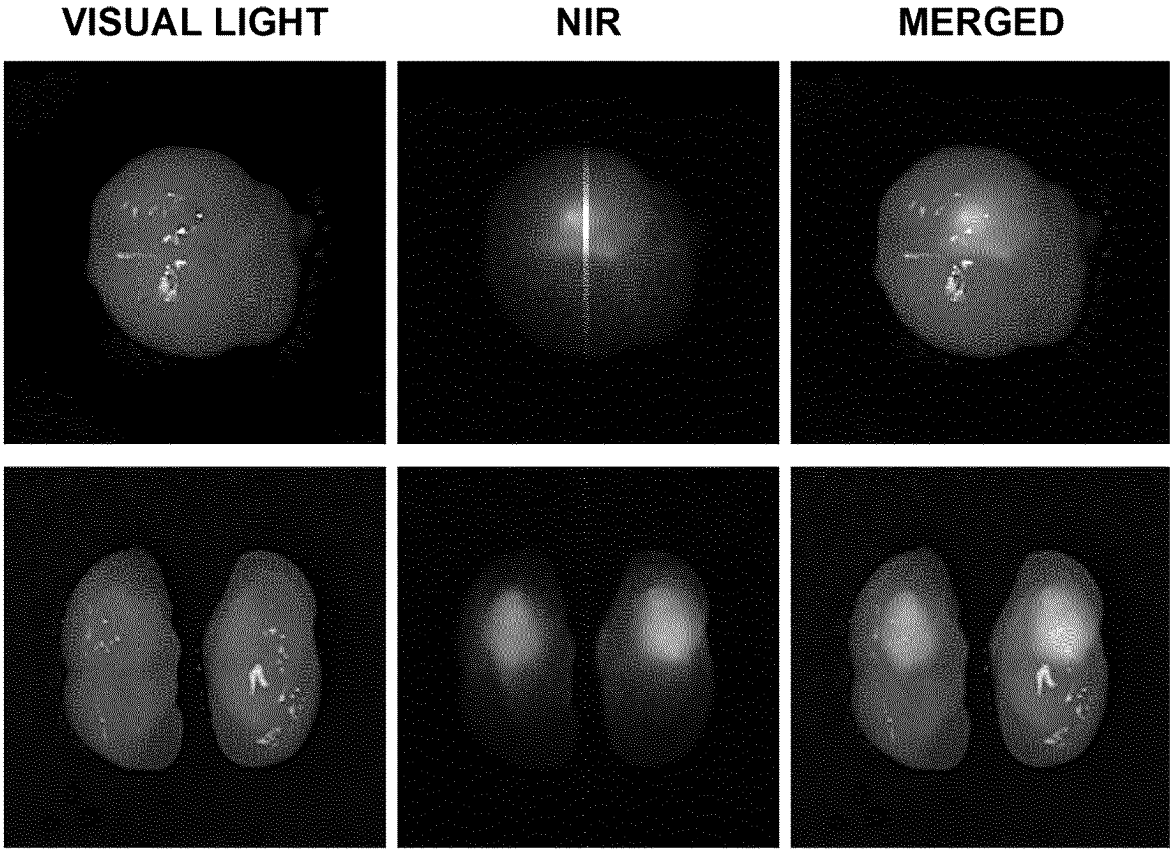


Fig. 6

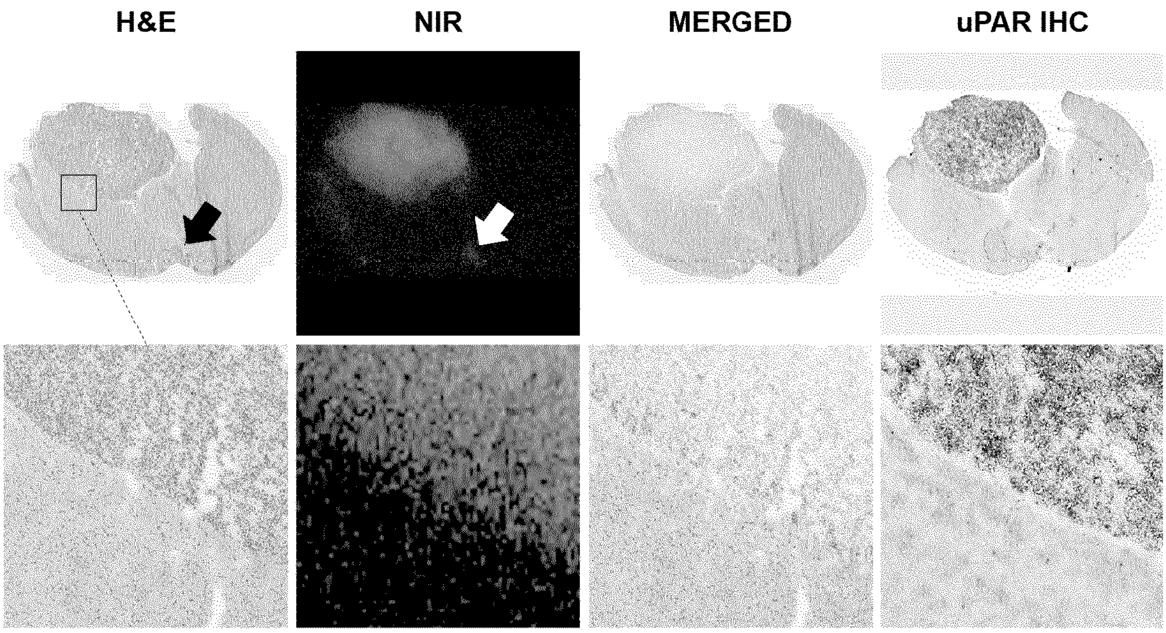

Fig. 7

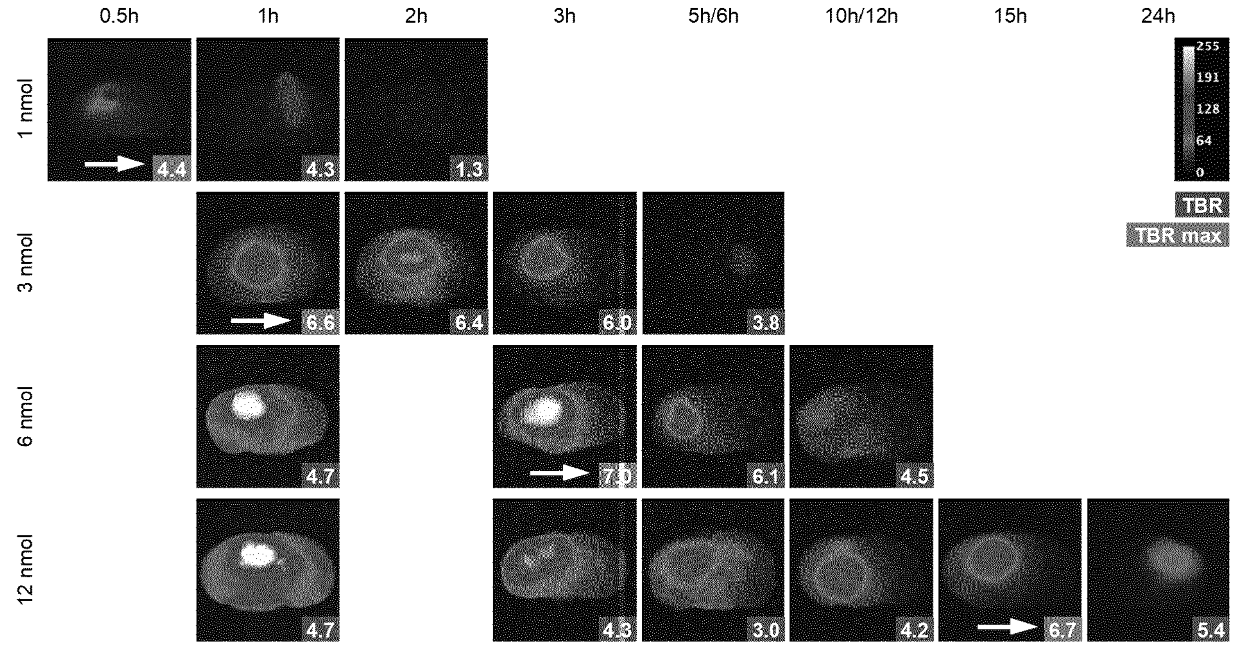
0.5h
1h
2h
3h
5h/6h
10h/12h
15h
24h
1 nmol
3 nmol
6 nmol
12 nmol
4.4
4.3
1.3
6.6
6.4
6.0
3.8
4.7
7.0
6.1
4.5
4.7
4.3
3.0
4.2
6.7
5.4
255
191
128
64
0
TBR
TBR max

Fig. 8

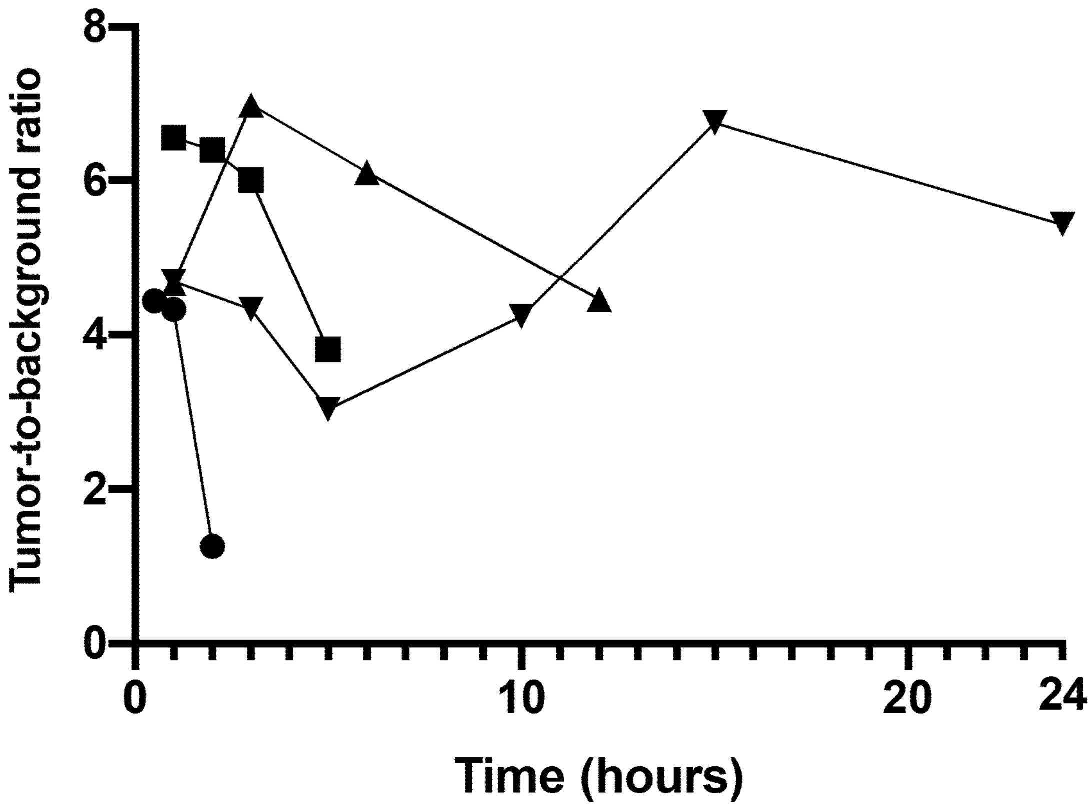
Tumor-to-background ratio
0
2
4
6
8
0
10
20
24
Time (hours)

Fig. 9

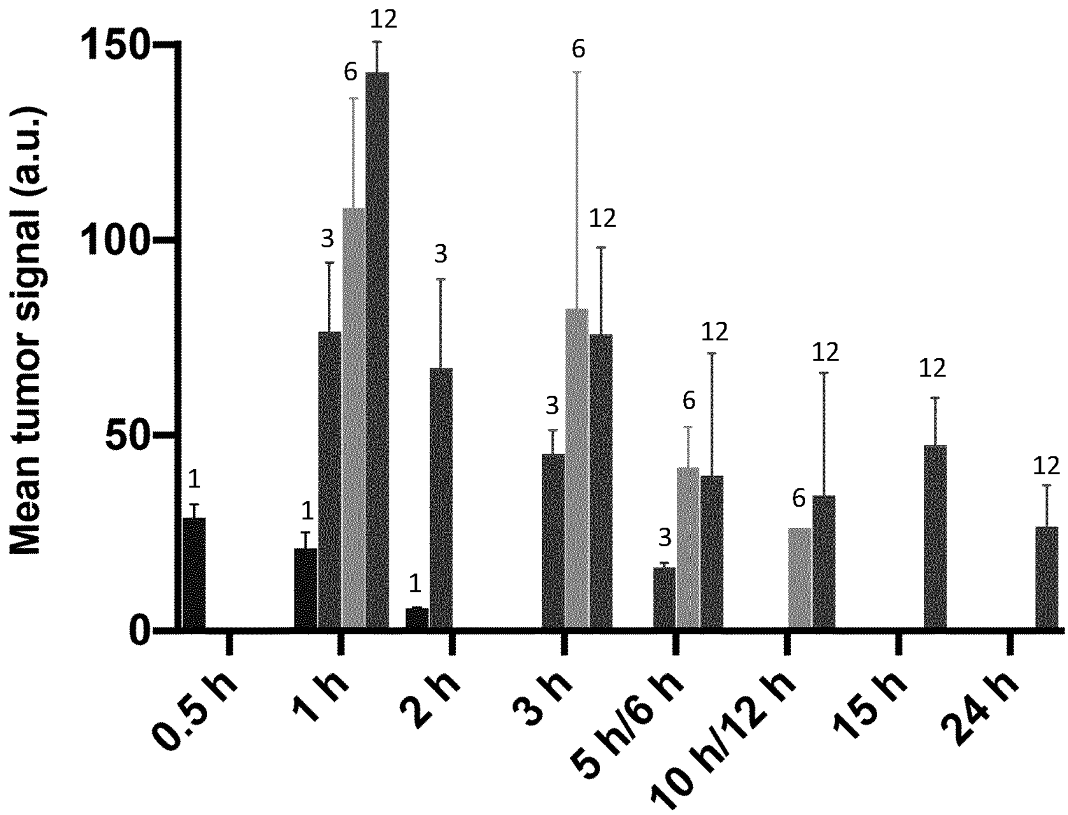
Mean tumor signal (a.u.)
150
100
50
0
1
1
6
12
3
1
3
6
12
3
3
6
12
12
6
12
12
0.5 h
1 h
2 h
3 h
5 h/6 h
10 h/12 h
15 h
24 h

Fig. 10

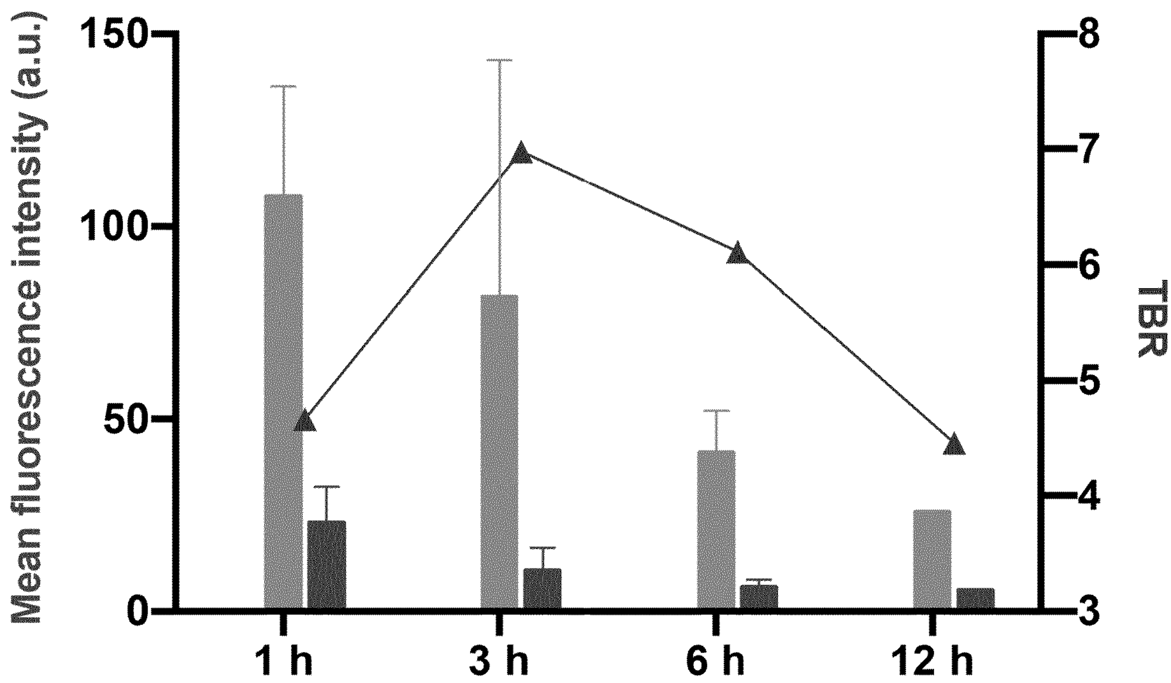
Mean fluorescence intensity (a.u.)
150
100
50
0
8
7
6
5
4
3
TBR
1 h
3 h
6 h
12 h

Fig. 11

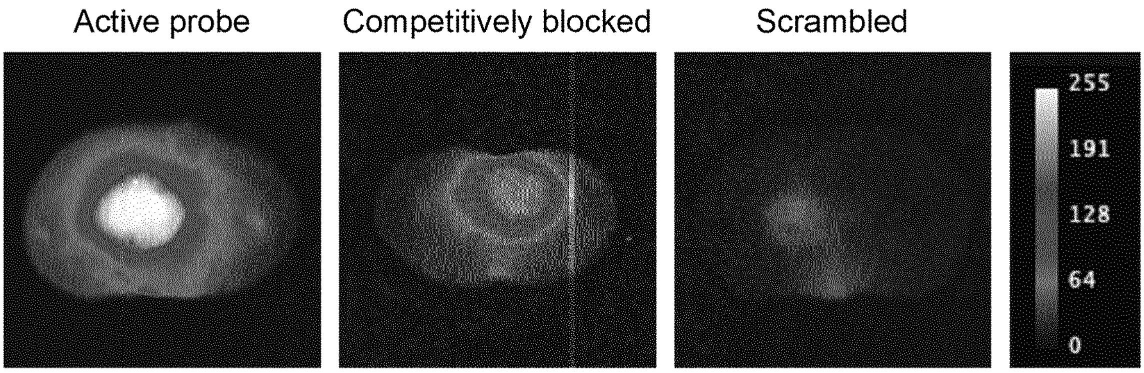
Active probe
Competitively blocked
Scrambled
255
191
128
64
0

Fig. 12

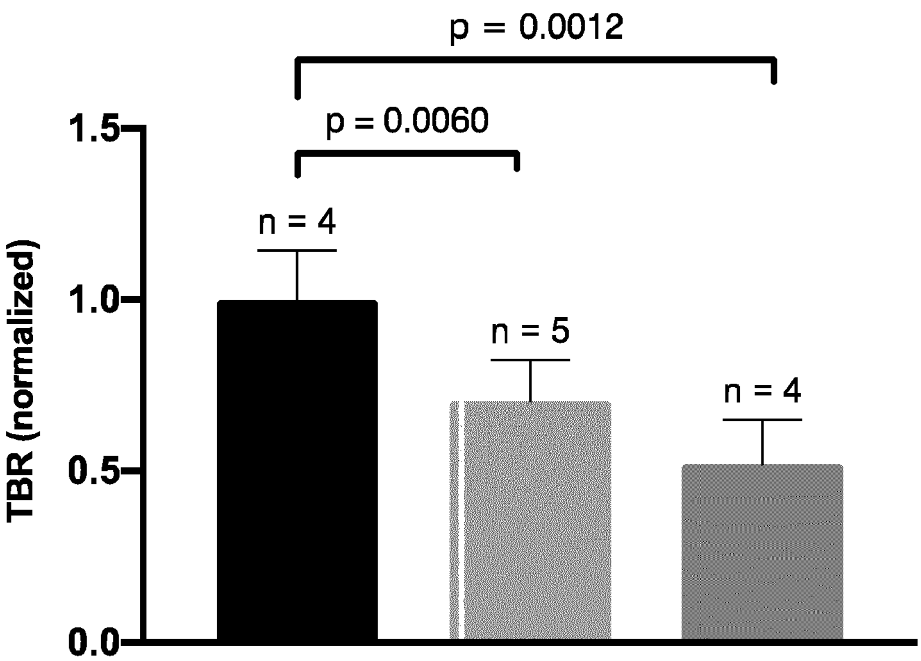
p = 0.0012
p = 0.0060
1.5
1.0
0.5
0.0
TBR (normalized)
n = 4
n = 5
n = 4

Fig. 13

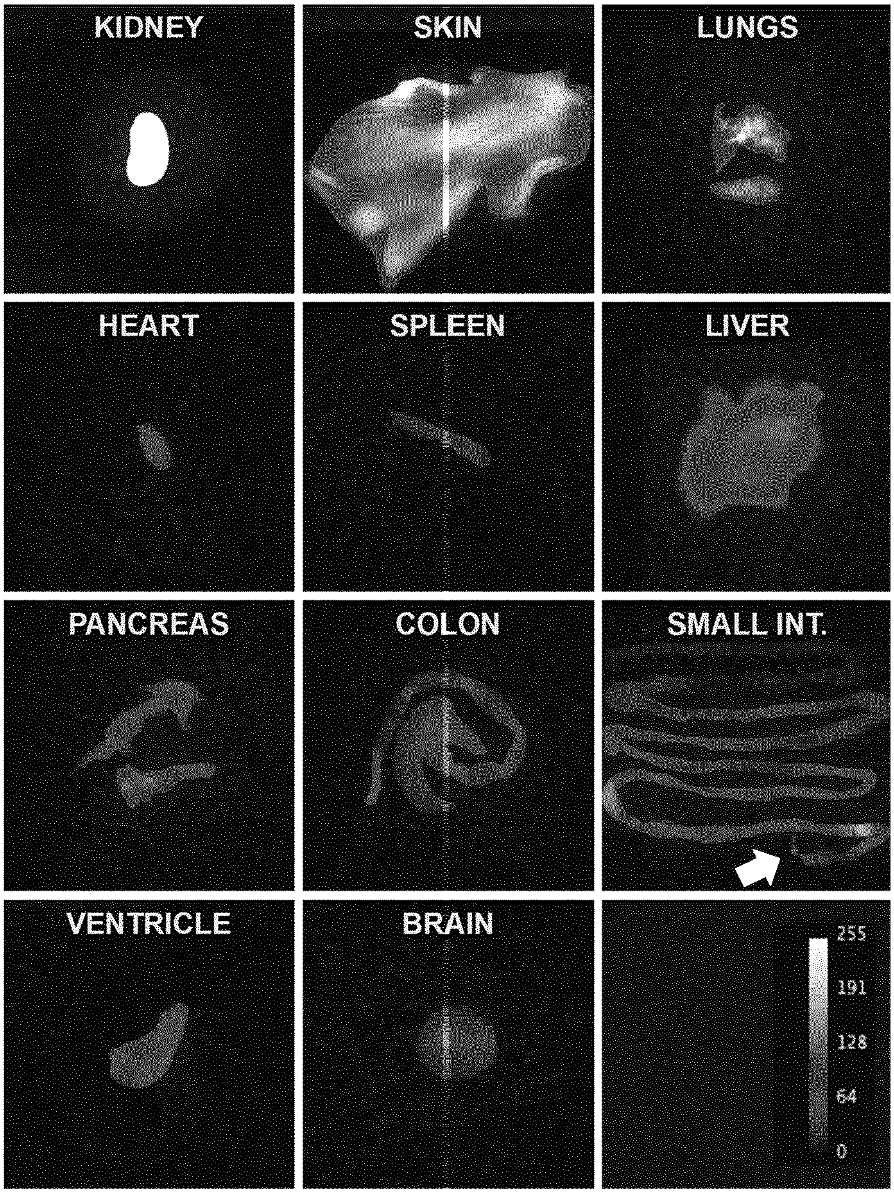
KIDNEY
SKIN
LUNGS
HEART
SPLEEN
LIVER
PANCREAS
COLON
SMALL INT.
VENTRICLE
BRAIN
255
191
128
64
0

Fig. 14

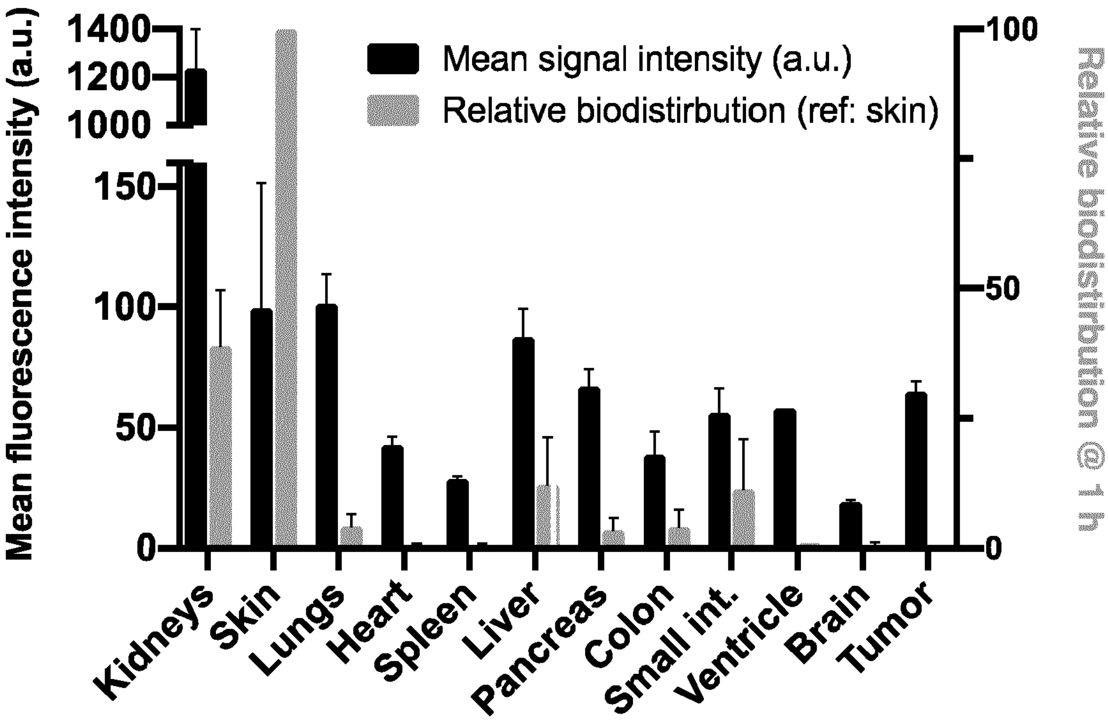
Mean fluorescence intensity (a.u.)
1400
1200
1000
150
100
50
0
Mean signal intensity (a.u.)
Relative biodistirbution (ref: skin)
100
50
0
Relative biodistirbution @ 1 h
Kidneys
Skin
Lungs
Heart
Spleen
Liver
Pancreas
Colon
Small int.
Ventricle
Brain
Tumor

Fig. 15

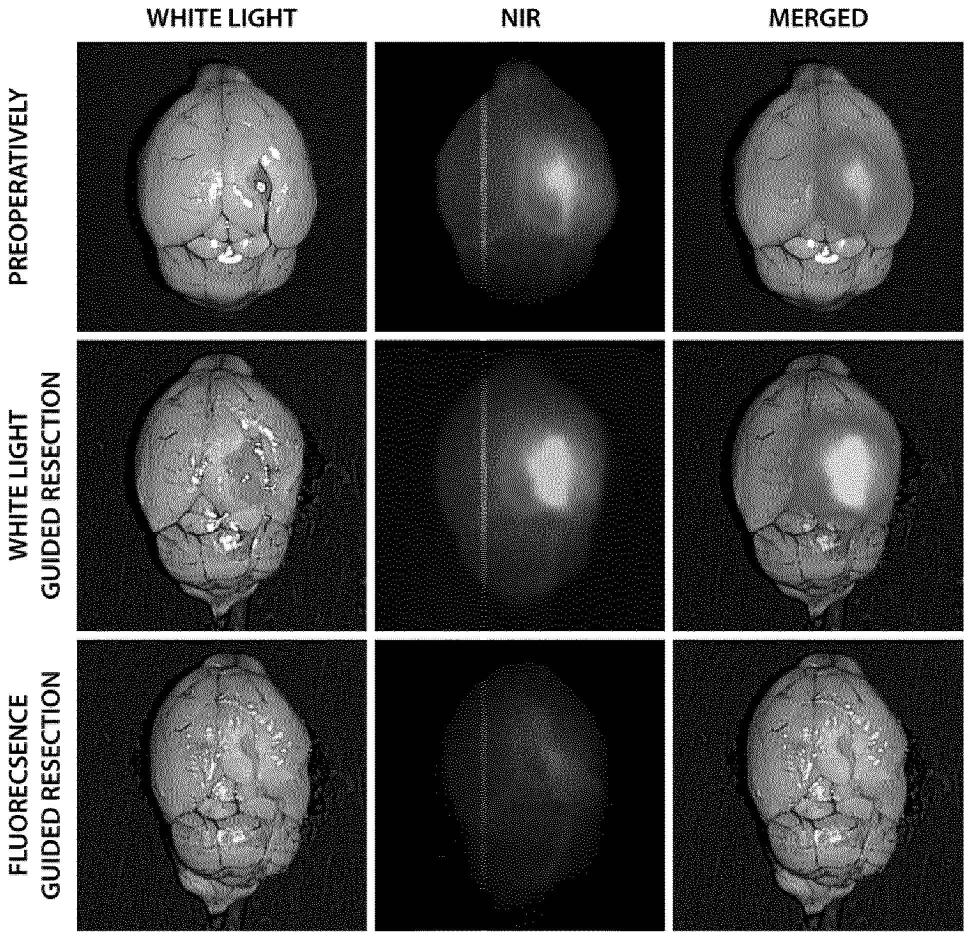
WHITE LIGHT
NIR
MERGED
PREOPERATIVELY
WHITE LIGHT GUIDED RESECTION
FLUORECSENCE GUIDED RESECTION

Fig. 16

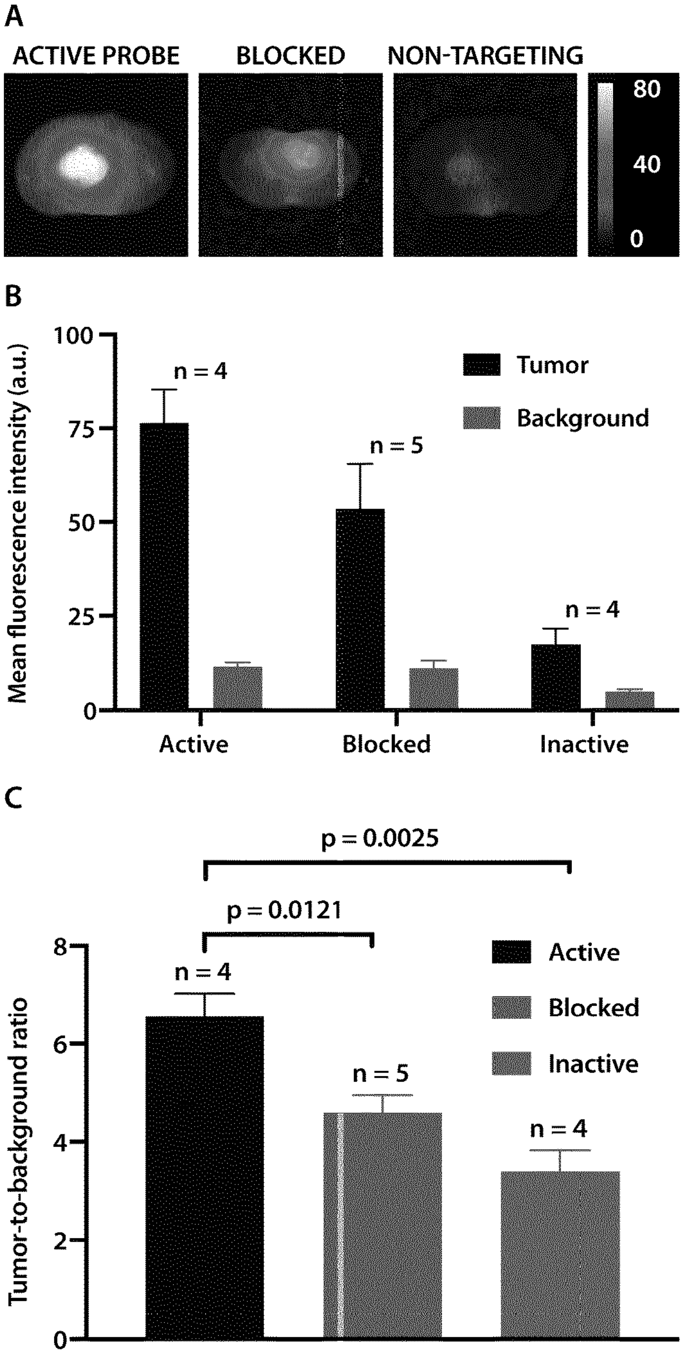
A
ACTIVE PROBE
BLOCKED
NON-TARGETING
80
40
0
B
Mean fluorescence intensity (a.u.)
100
75
50
25
0
n = 4
n = 5
n = 4
Tumor
Background
Active
Blocked
Inactive
C
p = 0.0025
p = 0.0121
Tumor-to-background ratio
8
6
4
2
0
n = 4
n = 5
n = 4
Active
Blocked
Inactive

Fig. 17

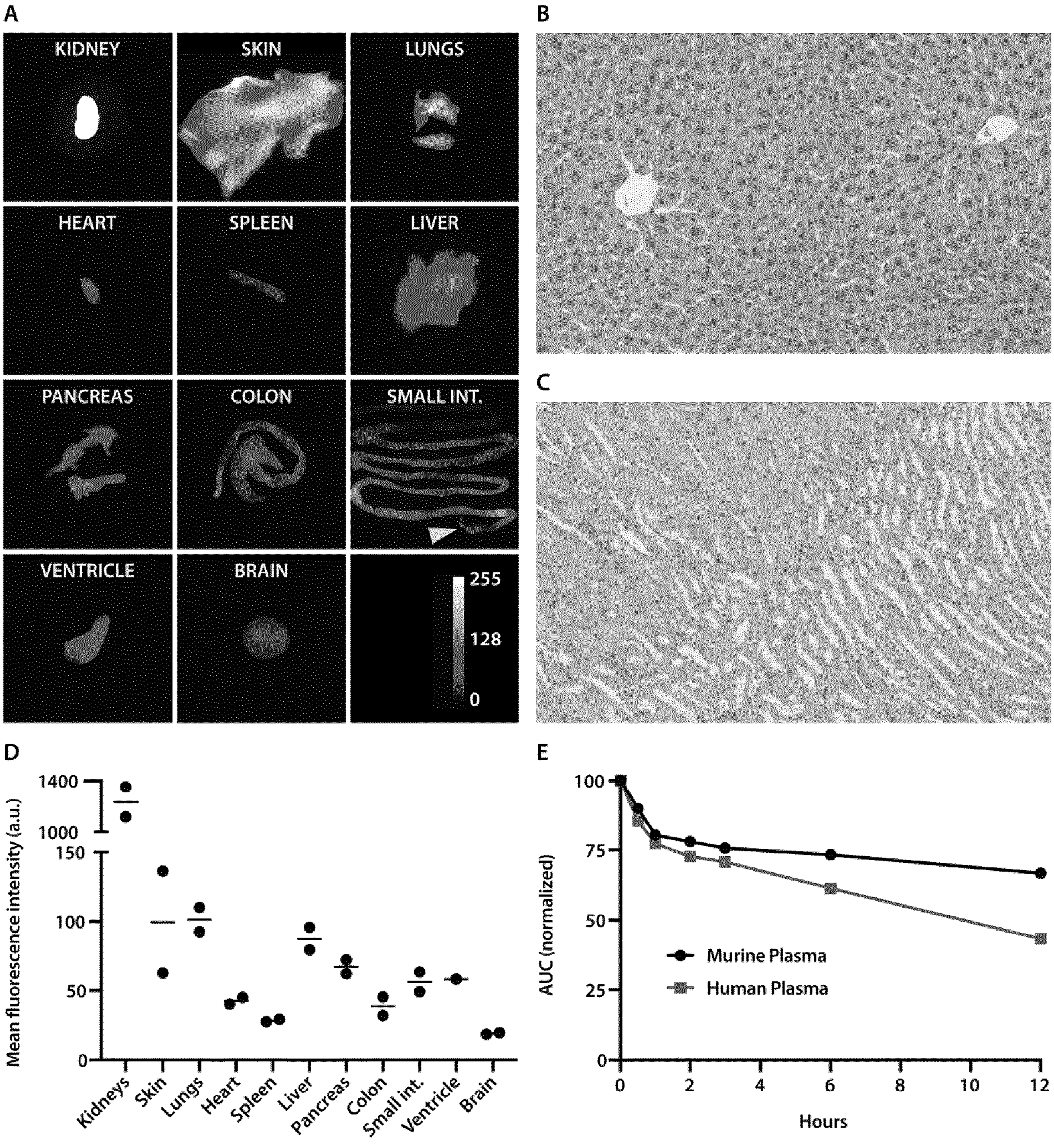
A
B
KIDNEY
SKIN
LUNGS
HEART
SPLEEN
LIVER
C
PANCREAS
COLON
SMALL INT.
VENTRICLE
BRAIN
255
128
0
D
E
Mean fluorescence intensity (a.u.)
1400
1000
150
100
50
0
Kidneys
Skin
Lungs
Heart
Spleen
Liver
Pancreas
Colon
Small int.
Ventricle
Brain
AUC (normalized)
100
75
50
25
0
Murine Plasma
Human Plasma
0
2
4
6
8
10
12
Hours

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016041558 A